# EUROPEAN PATENT APPLICATION

(11) **EP 4 725 477 A1**
(43) Date of publication of application: **15.04.2026**
(21) Application number: 25202817.0
(22) Date of filing: 17.09.2025
(51) Int. Cl.: A61K 9/16, A61K 36/185, A61K 39/395

(54) **PARTICLES, METHOD FOR PRODUCING PARTICLES, AND PHARMACEUTICAL COMPOSITION**

(30) Priority: 18.09.2024 JP 2024161286; 29.10.2024 JP 2024189535
(71) Applicant: ETRIA Co., Ltd., Yokohama, Kanagawa 220-0011 (JP)
(72) Inventor: Sato, Yuichi, Yokohama, 220-0011 (JP); Inoue, Ryota, Yokohama, 220-0011 (JP)
(74) Representative: Marks & Clerk LLP

(57) **Abstract**

A method for producing particles is provided. The method includes: preparing a bioactive-substance-containing liquid that includes a bioactive substance and a solvent; discharging the bioactive-substance-containing liquid as droplets; and removing the solvent from the droplets to form particles, wherein a biological activity ratio represented by {(biological activity level B/biological activity level A) × 100} is 80% or greater, where the biological activity level A is a biological activity level of the bioactive substance before the preparing the bioactive-substance-containing liquid, and the biological activity level B is a biological activity level of the bioactive substance after the preparing of the bioactive-substance-containing liquid.

## Description

### BACKGROUND OF THE INVENTION

### 1. Field of the Invention

The disclosures herein relate to particles, methods for producing particles, and pharmaceutical compositions.

### 2. Description of the Related Art

In recent years, studies on a drug delivery system, which is a technique for efficiently and safely administering an active pharmaceutical ingredient to an affected site, have been actively conducted. As part of the above trends, there is an increasing demand for fine particles for delivering an active pharmaceutical ingredient into a blood vessel. Such particles have a particle size of several hundred nanometers or less, and contain or carry a bioactive substance, such as the active pharmaceutical ingredient.

Examples of a production method for fine particles in which a bioactive substance is dispersed in each particle include emulsion solvent diffusion (ESD) and spray drying. The ESD method is a method in which a liquid including an organic polymer, a bioactive substance, and a good solvent is stirred while diffusing the liquid into a poor solvent to produce fine particles. The spray drying is a method in which a liquid including an organic polymer and a bioactive substance is sprayed, and heated to dry, thereby producing fine particles.

A powder is disclosed, for example, in Japanese Unexamined Patent Application Publication No. H11-114027. The disclosed powder is obtained by spray-drying a water-soluble substance selected from peptides, proteins, glycoproteins, saccharides, and nucleic acids.

Particles are disclosed, for example, in Japanese Unexamined Patent Application Publication No. 2021-28305. In each of the disclosed particles, a bioactive substance is dispersed in a base material.

### SUMMARY OF THE INVENTION

The present disclosure aims to provide a method for producing particles, in which a reduction in a biological activity level during production of the particles can be minimized.

According to one aspect of the present disclosure, a method for producing particles includes: preparing a bioactive-substance-containing liquid that includes a bioactive substance and a solvent; discharging the bioactive-substance-containing liquid as droplets; and removing the solvent from the droplets to form particles, wherein a biological activity ratio represented by {(biological activity level B/biological activity level A) × 100} is 80% or greater, where the biological activity level A is a biological activity level of the bioactive substance before the preparing the bioactive-substance-containing liquid, and the biological activity level B is a biological activity level of the bioactive substance after the preparing of the bioactive-substance-containing liquid.

As described above, the present disclosure can provide a method for producing particles, in which a reduction in a biological activity level during production of the particles can be minimized.

### BRIEF DESCRIPTION OF THE DRAWINGS

Fig. 1 is a schematic view illustrating one example of a particle production apparatus of a first embodiment of the present disclosure:
Fig. 2 is a schematic cross-sectional view illustrating one example of a liquid column resonance droplet discharger 3021, which serves as a discharger 302 in the particle production apparatus 300 of the first embodiment of the present disclosure:
Fig. 3 is a schematic cross-sectional view illustrating one example of a discharger 3022 using Rayleigh breakup, which serves as discharger 302 in the particle production apparatus 300 of the first embodiment of the present disclosure;
Fig. 4 is a schematic view illustrating another example of the particle production apparatus of the first embodiment of the present disclosure;
Fig. 5 is a schematic view illustrating an example of a particle production apparatus 400 of a second embodiment of the present disclosure; and
Fig. 6 is a schematic view illustrating an example of a particle production apparatus 500 of a third embodiment of the present disclosure.

### DESCRIPTION OF THE EMBODIMENTS

The technology of the relative art, which includes the inventions disclosed in Japanese Unexamined Patent Application Publication Nos. H11-114027 and 2021-28305, had the problems such that a biological activity of a bioactive substance is inactivated by heating, shaking, or stirring performed during a production process of particles in which the bioactive substance is included in a base material, and the bioactive substance is eluted into a solvent. In the technology of the related art, there are therefore concerns that the bioactive substance may not be encapsulated in each particle, or a biological activity level of the particles may be lowered.

The method for producing the particles of the present disclosure can sufficiently eliminate the above concerns in the related art. More specifically, the present disclosure can realize a particle production method that can minimize a reduction in a biological activity level during production of particles.

The details of the present disclosure will be described hereinafter.

### (Method for producing particles and particle production apparatus)

The method for producing the particles of the present disclosure includes: preparing a bioactive-substance-containing liquid that includes a bioactive substance and a solvent (bioactive-substance-containing liquid preparation step); discharging the bioactive-substance-containing liquid as droplets (discharging step); and removing the solvent from the droplets to form particles (particle formation step). The method for producing the particles of the present disclosure may further include other steps, as necessary.

A particle production apparatus associated with the present disclosure includes a discharger configured to discharge the bioactive-substance-containing liquid as droplets, and a particle forming device configured to remove the solvent from the droplets to form particles. The particle production apparatus may further include a bioactive-substance-containing liquid storage container and other members or devices, as necessary.

The method for producing the particles can be suitably performed by the particle production apparatus; the discharging step can be suitably performed by the discharger; the particle formation step can be suitably performed by the particle forming device; and other steps can be suitably performed by the other members or devices.

In the method for producing the particles of the present disclosure, heat and external stress are preferably not applied to the bioactive-substance-containing liquid and the droplets of the bioactive-substance-containing liquid. In other words, in the method for producing the particles of the present disclosure, application of external stress (shaking or stirring) that changes a biological activity of the bioactive substance, and application of heat (heating) that changes the biological activity of the bioactive substance are preferably not performed on the bioactive-substance-containing liquid and the droplets of the bioactive-substance-containing liquid.

Although details will be described later, since the method for producing the particles of the present disclosure has the above configuration, even in the case where a bioactive substance whose biological activity is likely to be changed by application of heat or external stress is included as a material of the particles, a change in the biological activity of the bioactive substance can be inhibited, and a reduction in the biological activity level during the production of the particles can be minimized.

### <Bioactive-substance-containing liquid preparation step>

The bioactive-substance-containing liquid preparation step includes preparing a bioactive-substance-containing liquid that includes a bioactive substance and a solvent.

A method for preparing the bioactive-substance-containing liquid is not particularly limited, and may be appropriately selected according to the intended purpose. Examples of the method include, but are not limited to, a method in which a liquid mixture of various materials is passed through a filter to prepare a bioactive-substance-containing liquid.

### <<Bioactive-substance-containing liquid>>

The bioactive-substance-containing liquid includes a bioactive substance and a solvent, and may further include a base material, and other ingredients, as necessary.

### <<<Bioactive substance>>>

The bioactive substance is an active ingredient that is used to allow a living body to exhibit a physiological effect. In the present specification, the term "physiological effect" refers to an effect obtained by allowing the bioactive substance to exhibit a biological activity at a target site, for example, quantitatively or qualitatively, or both quantitatively and qualitatively changing or affecting a living body, a tissue, a cell, a protein, deoxyribonucleic acid (DNA), or ribonucleic acid (RNA).

In the present specification, the term "biological activity" refers to the bioactive substance acting on a target site (e.g., target tissues) to change or affect the target site. Examples of the target site include, but are not limited to, receptors present on surfaces of cells or inside cells. In this case, the biological activity of the bioactive substance is to bind to a specific receptor to transmit a signal to the cell, and as a result, a physiological effect is exhibited.

The bioactive substance may be a substance that is transformed into a mature form by an enzyme present inside a living body to bind to a specific receptor as the mature form, thereby exhibiting a physiological effect. In the present specification, a substance in the form before being transformed into a mature form is also considered as the bioactive substance.

The bioactive substance may be a substance produced by an organism (a human or organisms other than a human) or may be an artificially synthesized substance.

The bioactive substance may have a characteristic such that the biological activity of the bioactive substance is changed by heat, external stress, or both heat and external stress. In the case where heat and external stress are not applied to the bioactive-substance-containing liquid or droplets of the bioactive-substance-containing liquid in the method for producing the particles of the present disclosure, a reduction in a biological activity level during production of particles can be minimized by using the above bioactive substance.

In the present specification, the term "characteristic such that the biological activity of the bioactive substance is changed" refers to , for example, a characteristic that a biological activity level is increased or reduced, a characteristic that a biological activity efficiency is increased or reduced, and a characteristic that a type of a biological activity is changed. Among the above characteristics, the characteristic that the biological activity level is reduced or the characteristic that the biological activity efficiency is reduced is preferable, with the characteristic that the biological activity level is reduced being more preferable.

The characteristic that the type of the biological activity is changed may be reversible change, or irreversible change, but is preferably an irreversible change.

In the present specification, the term "biological activity level" refers to a measured value obtained by quantitatively measuring the biological activity of the bioactive substance. The "quantitatively measuring" is not limited to a method in which the biological activity level is directly quantitatively measured, and may be a relative quantitative measuring method.

In the present specification, the term "heating" means application of thermal energy to the bioactive substance (or the bioactive-substance-containing liquid). By "heating," a molecular structure or three-dimensional structure of the bioactive substance may be changed, which may cause a change in the biological activity. In the case where the bioactive substance is a protein, specific examples of the change include a thermal denaturation of the protein or a low temperature denaturation of the protein. In the case where the bioactive substance is a nucleic acid, examples of the change include decomposition of the nucleic acid.

A heating temperature is not particularly limited, and may be appropriately selected according to the bioactive substance for use.

In the present specification, the term "external stress" refers to a force that is externally applied to the bioactive substance (or the bioactive-substance-containing liquid). Examples of the external stress include, but are not limited to, shaking, stirring, and shear stress. When the external stress is applied, a molecular structure or three-dimensional structure of the bioactive substance may be changed, which may cause a change in the biological activity. In the case where the bioactive substance is a protein, specific examples of the change include inactivation of the protein due to the change in the higher-order structure. Examples of the protein that is likely to be inactivated by external stress include, but are not limited to, proteins (e.g., enzymes and antibodies) that form multimers.

A process that generates external stress is not particularly limited, and may be appropriately selected according to the bioactive substance for use. Examples of the process include, but are not limited to, shaking, stirring, pulverizing, an ultrasonic treatment, a homogenizer treatment, and spraying.

The bioactive substance is not particularly limited, and may be appropriately selected according to the intended purpose. Examples of the bioactive substance include, but are not limited to, a bioactive substance included in a pharmaceutical composition, a bioactive substance included in a functional food, a bioactive substance included in functional cosmetics, and the like.

The above-listed bioactive substances may be used alone or in combination.

### -Bioactive substance included in pharmaceutical composition-

The bioactive substance included in the pharmaceutical composition is not particularly limited, and may be appropriately selected according to the intended purpose. Examples of the bioactive substance included in the pharmaceutical composition include, but are not limited to, nucleic acids, polypeptides (including proteins), saccharides (carbohydrates), lipids, and low-molecular-weight compounds.

The above-listed bioactive substances may be used alone or in combination.

### --Nucleic acids--

Typical examples of the nucleic acids include DNA, RNA, and a combination of DNA and RNA. Moreover, the nucleic acids may be chemically-modified nucleic acids in which a part of a sequence or an entire sequence of a respective nucleic acid is chemically modified by substitution.

The nucleic acids also include chemically synthesized nucleic acid analogs, such as peptide nucleic acids (PNA) and morpholino antisense oligonucleotides.

In the case where suppression of expression of a target gene is desired, for example, the nucleic acid for use may be an antisense nucleic acid complementary to a transcription product of a target gene or part of the transcription product, a nucleic acid exhibiting a ribozyme activity that causes a specific cleavage of a transcription product of a target gene, a short-chain nucleic acid having a function of suppressing expression of a target gene due to RNAi interference, microRNA (miRNA), an aptamer, or a locked nucleic acid obtained by modifying an oligonucleotide.

### --Polypeptides--

The polypeptide is a polymer composed of a plurality of amino acids. Among polypeptides, a polypeptide having a higher-order structure and exhibiting a function owing to the higher-order structure is referred to as a protein.

The polypeptides may be optionally modified.

The modification is not particularly limited, and may be appropriately selected according to the intended purpose. Examples of the modification include, but are not limited to, acylation, ADP-ribosylation, amidation, a covalent bond of flavin, a covalent bond of a heme moiety, a covalent bond of a nucleotide or a nucleotide derivative, a covalent bond of a lipid or a lipid derivative, a covalent bond of phosphatidylinositol, crosslink, cyclization, formation of a disulfide bond, demethylation, formation of covalent crosslink, formation of cystine, formation of pyroglutamate, formylation, γ-carboxylation, glycosylation, formation of a GPI anchor, hydroxylation, iodination, methylation, myristoylation, oxidation, a protein decomposition treatment, phosphorylation, prenylation, racemization, selenoylation, sulfation, tRNA mediation addition of amino acid to protein (e.g., arginylation), and ubiquitination.

In the case where inhibition or suppression of a function of a target protein is desired, examples of the protein include a target protein variant that is dominant-negative to the target protein, and an antibody that binds to a target protein.

The antibody may be a polyclonal antibody or a monoclonal antibody, as long as the antibody binds to a target protein. The antibody may be a polyspecific antibody, such as a bispecific antibody and a trispecific antibody.

The antibody may be derived from any animal as long as the antibody exhibits a physiological effect, but the antibody is preferably a human antibody, a human chimeric antibody, or a humanized antibody.

Examples of the antibody include, but are not limited to, immunoglobulin molecules, such as IgG, IgE, IgM, IgA, and IgD. The antibody includes an antibody fragment having an antigen binding region (e.g., F(ab')2 fragments, Fab' fragments, Fab fragments, Fv fragments, rIgG fragments, and a single chain antibody) and a modified antibody (e.g., a labeled antibody), as long as the antibody can bind to a specific antigen.

Examples of the protein also include an enzyme.

The enzyme is not particularly limited, and may be appropriately selected according to the intended purpose. Examples of the enzyme include, but are not limited to, hydrolase, phosphorylase, dephosphorylase, transferase, oxidoreductase, lyase, isomerase, and synthesized enzyme.

Specific examples of the protein include, but are not limited to, quercetin, testosterone, indomethacin, tranilast, and tacrolimus.

### --Saccharides--

The saccharides are not particularly limited, and may be appropriately selected according to the intended purpose. Examples of the saccharides include, but are not limited to, monosaccharides, disaccharides, oligosaccharides, and polysaccharides. Moreover, the saccharides also include a complex saccharide, in which a saccharide is bonded to a protein or a lipid via a covalent bond, or a glycoside in which aglycone, such as alcohol, phenol, saponin, or a dye, is bonded to a reducing group of a saccharide.

### --Lipids--

The lipids are not particularly limited, and may be appropriately selected according to the intended purpose. Examples of the lipids include, but are not limited to, simple lipids, complex lipids, and derived lipids.

### --Low-molecular-weight compounds--

The low-molecular-weight compounds typically include substances having a molecular weight of several hundreds to several thousands.

As the low-molecular-weight compounds, there are sparingly water-soluble substances and water-soluble substances. The low-molecular-weight compounds may be in the form of salts or hydrates as long as the low-molecular-weight compounds function as the bioactive substance.

The sparingly water-soluble substances are not particularly limited, and may be appropriately selected according to the intended purpose. Examples of the sparingly water-soluble substances include, but are not limited to, griseofulvin, itraconazole, norfloxacin, tamoxifen, cyclosporine, glibenclamide, troglitazone, nifedipine, phenacetin, phenytoin, digitoxin, nilvadipine, diazepam, chloramphenicol, indomethacin, nimodipine, dihydroergotoxine, cortisone, dexamethasone, naproxen, tulobuterol, beclometasone dipropionate, fluticasone propionate, pranlukast, tranilast, loratadine, tacrolimus, amprenavir, bexarotene, calcitriol, clofazimine, digoxin, doxercalciferol, dronabinol, etoposide, isotretinoin, lopinavir, ritonavir, progesterone, saquinavir, sirolimus, tretinoin, amphotericin, fenoldopam, melphalan, paricalcitol, propofol, voriconazole, ziprasidone, docetaxel, haloperidol, lorazepam, teniposide, testosterone, and valrubicin.

The above-listed sparingly water-soluble substances may be used alone or in combination.

The water-soluble substances are not particularly limited, and may be appropriately selected according to the intended purpose. Examples of the water-soluble substances include, but are not limited to, abacavir, acetaminophen, acyclovir, amiloride, amitriptyline, antipyrine, atropine, buspirone, caffeine, captopril, chloroquine, chlorpheniramine, cyclophosphamide, diclofenac, desipramine, diazepam, diltiazem, diphenhydramine, disopyramide, doxine, doxycycline, enalapril, ephedrine, ethambutol, ethinylestradiol, fluoxetine, imipramine, glucose, ketorol, ketoprofen, labetalol, levodopa, levofloxacin, metoprolol, metronidazole, midazolam, minocycline, misoprostol, metformin, nifedipine, phenobarbital, prednisolone, promazine, propranolol, quinidine, rosiglitazone, salicylic acid, theophylline, valproic acid, verapamil, and zidovudine.

The above-listed water-soluble substances may be used alone or in combination.

Specific examples of the low-molecular-weight compounds include, but are not limited to, kinase inhibitors, such as gefitinib, erlotinib, osimertinib, bosutinib, vandetanib, alectinib, lorlatinib, abemaciclib, tyrphostin AG494, sorafenib, dasatinib, lapatinib, imatinib, motesanib, lestaurtinib, tandutinib, dorsomorphin, axitinib, and 4-benzyl-2-methyl-1,2,4-thiadiazolidine-3,5-dione.

### -Bioactive substance included in functional food-

The bioactive substance included in the functional food is not particularly limited, and may be appropriately selected according to the intended purpose. Examples of the bioactive substance included in the functional food include, but are not limited to, vitamin A, vitamin D, vitamin E, lutein, zeaxanthin, lipoic acid, flavonoid, and fatty acids.

The above-listed bioactive substances may be used alone or in combination.

Examples of the fatty acids include, but are not limited to, omega-3 fatty acids, and omega-6 fatty acids.

### -Bioactive substance included in functional cosmetics-

The bioactive substance included in the functional cosmetics is not particularly limited, and may be appropriately selected according to the intended purpose. Examples of the bioactive substance included in the functional cosmetics include, but are not limited to: alcohols; fatty alcohols; polyols; aldehydes; alkanol amines; alkoxylated alcohols (e.g., polyethylene glycol derivatives of alcohols or fatty alcohols); alkoxylated amides; alkoxylated amines; alkoxylated carboxylic acids; salt-containing amides (e.g., ceramides); amines; amino acids, salts and alkyl-substituted derivatives of amino acids; alkyl-substituents and acyl derivatives; polyacrylic acids; acrylamide copolymers; adipic acid copolymers; amino silicones; biological polymers and derivatives of biological polymers; butylene copolymers; carbohydrates (e.g., polysaccharides, chitosan, and derivatives of the foregoing); carboxylic acids, carbomers; esters; ethers; polymer ethers (e.g., PEG derivatives and PPG derivatives); glyceryl esters and derivatives of glyceryl esters; halogen compounds; heterocyclic compounds and salts of heterocyclic compounds; hydrophilic colloids and derivatives of hydrophilic colloids, including salts and rubber (e.g., cellulose derivatives, gelatin, xanthan gum, and natural rubber); imidazolines; inorganic materials (e.g., clay, TiO₂, and ZnO); ketones (e.g., camphor); isethionates; lanolins and derivatives of lanolins; organic salts; phenols (e.g., parabens) and salts of phenols; phosphorus compounds (e.g., phosphate derivatives); polyacrylates and acrylate copolymers; proteins and enzyme derivatives (e.g., collagen); synthetic polymers and salts of synthetic polymers; siloxanes and silanes; sorbitan derivatives; sterols; sulfonic acids and derivatives of sulfonic acids; and wax.

The above-listed bioactive substances may be used alone or in combination.

The bioactive substance is preferably a bioactive substance included in a pharmaceutical composition, more preferably a protein, a nucleic acid, or both a protein and a nucleic acid, and yet more preferably an antibody, an enzyme, or both an antibody and an enzyme.

An amount of the bioactive substance is not particularly limited, and may be appropriately selected according to the intended purpose. The amount of the bioactive substance is preferably 1 percent by mass or greater and 100 percent by mass or less, and more preferably 25 percent by mass or greater and 99 percent by mass or less, relative to a total amount of the particles.

When the amount of the bioactive substance is 25 percent by mass or greater relative to the total amount of the particles, sustained-release particles that can stably release the bioactive substance over a long period can be obtained.

When the amount of the bioactive substance is 95 percent by mass or greater relative to a total amount of the particles, a drug dose per administration can be increased so that the number of administrations can be reduced.

The amount of the bioactive substance included in the particles can be controlled by adjusting the formulation of the bioactive-substance-containing liquid when the particles are produced.

### <<<Base material>>>

The base material is a material that is a base constituting the particles. The base material is preferably a solid at room temperature.

The base material is not particularly limited, as long as the base material is not a material that adversely affects the bioactive substance. The base material may be appropriately selected according to the intended purpose. The base material may be a low-molecular-weight substance or a high-molecular-weight substance.

### -Low-molecular-weight substance-

The low-molecular-weight substance is preferably a compound having a weight average molecular weight of less than 15,000.

The low-molecular-weight substance is not particularly limited, and may be appropriately selected according to the intended purpose. Examples of the low-molecular-weight substance include, but are not limited to, lipids, saccharides (carbohydrates), cyclodextrins, amino acids, and organic acids.

The above-listed low-molecular-weight substances may be used alone or in combination.

### --Lipids--

The lipids are not particularly limited, and may be appropriately selected according to the intended purpose. Examples of the lipids include, but are not limited to, medium or long chain monoglyceride, medium or long chain diglyceride, medium or long chain triglyceride, phospholipid, vegetable oil (e.g., soybean oil, avocado oil, squalene oil, sesame oil, olive oil, corn oil, rapeseed oil, safflower oil, and sunflower oil), fish oil, seasoning oil, water-insoluble vitamins, fatty acids, medium-chain triglyceride, mixtures of the foregoing, and derivatives of the foregoing.

The above-listed lipids may be used alone or in combination.

### --Saccharides--

The saccharides are not particularly limited, and may be appropriately selected according to the intended purpose. Examples of the saccharides include, but are not limited to: monosaccharides and polysaccharides, such as glucose, mannose, idose, galactose, fucose, ribose, xylose, lactose, sucrose, maltose, trehalose, turanose, raffinose, maltotriose, acarbose, cyclodextrins, amylose (starch), and cellulose; sugar alcohols (polyols), such as glycerin, sorbitol, lactitol, maltitol, mannitol, xylitol, and erythritol; and derivatives of the foregoing.

The above-listed saccharides may be used alone or in combination.

### --Cyclodextrins--

The cyclodextrins are not particularly limited, and may be appropriately selected according to the intended purpose. Examples of the cyclodextrins include, but are not limited to, hydroxypropyl-β-cyclodextrin, β-cyclodextrin, γ-cyclodextrin, α-cyclodextrin, and cyclodextrin derivative.

The above-listed cyclodextrins may be used alone or in combination.

### --Amino acids--

The amino acids are not particularly limited, and may be appropriately selected according to the intended purpose. Examples of the amino acids include, but are not limited to, valine, lysine, leucine, threonine, isoleucine, asparagine, glutamine, phenylalanine, aspartic acid, serine, glutamic acid, methionine, arginine, glycine, alanine, tyrosine, proline, histidine, cysteine, tryptophan, and derivatives of the foregoing.

The above-listed amino acids may be used alone or in combination.

### --Organic acids--

The organic acids are not particularly limited, and may be appropriately selected according to the intended purpose. Examples of the organic acids include, but are not limited to, adipic acid, ascorbic acid, citric acid, fumaric acid, gallic acid, glutaric acid, lactic acid, malic acid, maleic acid, succinic acid, tartaric acid, and derivatives of the foregoing.

The above-listed organic acids may be used alone or in combination.

### -High-molecular-weight substance-

The high-molecular-weight substance is preferably a compound having a weight average molecular weight of 15,000 or greater.

The high-molecular-weight substance is not particularly limited, and may be appropriately selected according to the intended purpose. Examples of the high-molecular-weight substance include, but are not limited to, water-soluble cellulose, polyalkylene glycol, poly(meth)acrylamide, poly(meth)acrylic acid, poly(meth)acrylate, polyallylamine, polyvinyl pyrrolidone, polyvinyl alcohol, polyvinyl acetate, biodegradable polyesters, polyglycolic acid, polyamino acid, gelatin, protein (e.g., fibrin), polysaccharides, and derivatives of the foregoing.

The above-listed high-molecular-weight substances may be used alone or in combination.

### --Water-soluble cellulose--

The water-soluble cellulose is not particularly limited, and may be appropriately selected according to the intended purpose. Examples of the water-soluble cellulose include, but are not limited to, alkyl cellulose (e.g., methyl cellulose and ethyl cellulose), hydroxyalkyl cellulose (e.g., hydroxyethyl cellulose and hydroxypropyl cellulose), and hydroxyalkyl alkyl cellulose (e.g., hydroxyethyl methyl cellulose and hydroxypropyl methyl cellulose). Among the above-listed water-soluble celluloses, hydroxypropyl cellulose and hydroxypropyl methyl cellulose are preferable, with hydroxypropyl cellulose being more preferable, because of high biocompatibility and high solubility to a solvent used in production of particles.

The above-listed water-soluble celluloses may be used alone or in combination.

A viscosity of the 2 percent by mass aqueous solution of hydroxypropyl cellulose at 20°C is not particularly limited, and may be appropriately selected according to the intended purpose. The viscosity of the 2 percent by mass aqueous solution of hydroxypropyl cellulose at 20°C is preferably 2.0 mPa·s (centipoise (cps)) or greater and 4,000 mPa·s (centipoise (cps)) or less.

The viscosity of the hydroxypropyl cellulose is considered to depend on a weight average molecular weight and substitution degree of the hydroxypropyl cellulose. The weight average molecular weight of the hydroxypropyl cellulose is not particularly limited, and may be appropriately selected according to the intended purpose. The weight average molecular weight of the hydroxypropyl cellulose is preferably 15,000 or greater and 400,000 or less. The weight average molecular weight can be measured, for example, by gel permeation chromatography (GPC).

Commercial products of the hydroxypropyl cellulose are not particularly limited, and may be appropriately selected according to the intended purpose. Examples of the commercial products include, but are not limited to, HPC-SSL (molecular weight: 15,000 or greater and 30,000 or less, viscosity: 2.0 mPa·s or greater and 2.9 mPa·s or less), HPC-SL (molecular weight: 30,000 or greater and 50,000 or less, viscosity: 3.0 mPa·s or greater and 5.9 mPa·s or less), HPC-L (molecular weight: 55,000 or greater and 70,000 or less, viscosity: 6.0 mPa·s or greater and 10.0 mPa·s or less), HPC-M (molecular weight: 110,000 or greater and 150,000 or less, viscosity: 150 mPa·s or greater and 400 mPa·s or less), and HPC-H (molecular weight: 250,000 or greater and 400,000 or less, viscosity: 1,000 mPa·s or greater and 4,000 mPa·s or less) (all manufactured by Nippon Soda Co., Ltd.). Among the above-listed commercial products, HPC-SSL is preferable.

The above-listed commercial products may be used alone or in combination.

For each of the above commercial products, the molecular weight is measured by gel permeation chromatography (GPC), and the viscosity is measured in the form of a 2 percent by mass aqueous solution at 20°C.

An amount of the hydroxypropyl cellulose is not particularly limited, and may be appropriately selected according to the intended purpose. The amount of the hydroxypropyl cellulose is preferably 50 percent by mass or greater, more preferably 50 percent by mass or greater and 99 percent by mass or less, yet more preferably 75 percent by mass or greater and 99 percent by mass or less, and particularly preferably 80 percent by mass or greater and 99 percent by mass or less, relative to a total amount of the base material.

### --Polyalkylene glycol--

The polyalkylene glycol is not particularly limited, and may be appropriately selected according to the intended purpose. Examples of the polyalkylene glycol include, but are not limited to, polyethylene glycol (PEG), polypropylene glycol, polybutylene glycol, and copolymers of the foregoing.

The above-listed polyalkylene glycols may be used alone or in combination.

### --Poly(meth)acrylamide--

The poly(meth)acrylamide is not particularly limited, and may be appropriately selected according to the intended purpose. Examples of the poly(meth)acrylamide include, but are not limited to, polymers of monomers, such as N-methyl(meth)acrylamide, N-ethyl(meth)acrylamide, N-propyl(meth)acrylamide, N-butyl(meth)acrylamide, N-benzyl(meth)acrylamide, N-hydroxyethyl(meth)acrylamide, N-phenyl(meth)acrylamide, N-tolyl(meth)acrylamide, N-(hydroxyphenyl)(meth)acrylamide, N-(sulfamoylphenyl)(meth)acrylamide, N-(phenylsulfonyl)(meth)acrylamide, N-(tolylsulfonyl)(meth)acrylamide, N,N-dimethyl(meth)acrylamide, N-methyl N-phenyl(meth)acrylamide, and N-hydroxyethyl-N-methyl(meth)acrylamide.

Any of the above-listed monomers may be polymerized alone, or any combination of the above-listed monomers may be polymerized together. In addition, the above-listed polymers may be used alone or in combination.

### --Poly(meth)acrylic acid--

The poly(meth)acrylic acid is not particularly limited, and may be appropriately selected according to the intended purpose. Examples of the poly(meth)acrylic acid include, but are not limited to: homopolymers, such as polyacrylic acid and polymethacrylic acid; and copolymers, such as an acrylic acid-methacrylic copolymer.

The above-listed poly(meth)acrylic acids may be used alone or in combination.

### --Poly(meth)acrylate--

The poly(meth)acrylate is not particularly limited, and may be appropriately selected according to the intended purpose. Examples of the poly(meth)acrylate include, but are not limited to, polymers of monomers, such as ethylene glycol di(meth)acrylate, diethylene glycol di(meth)acrylate, propylene glycol di(meth)acrylate, glycerol poly(meth)acrylate, polyethylene glycol(meth)acrylate, trimethylolpropane tri(meth)acrylate, pentaerythritol tetra(meth)acrylate, and 1,3-butyleneglycol di(meth)acrylate.

Any of the above-listed monomers may be polymerized alone, or any combination of the above-listed monomers may be polymerized together. Moreover, the above-listed polymers may be used alone or in combination.

### --Polyallylamine--

The polyallylamine is not particularly limited, and may be appropriately selected according to the intended purpose. Examples of the polyallylamine include, but are not limited to, diallylamine and triallylamine.

The above-listed polyallylamines may be used alone or in combination.

### --Polyvinyl pyrrolidone--

The polyvinyl pyrrolidone is not particularly limited, and may be appropriately selected according to the intended purpose.

As the polyvinyl pyrrolidone, an appropriately synthesized product may be used, or a commercial product may be used. Examples of the commercial product of the polyvinyl pyrrolidone include, but are not limited to, PLASDONE C-15 (available from ISP TECHNOLOGIES); KOLLIDON VA 64, KOLLIDON K-30, and KOLLIDON CL-M (all available from KAWARLAL); and KOLLICOAT IR (available from BASF).

The above-listed polyvinyl pyrrolidones may be used alone or in combination.

### --Polyvinyl alcohol--

The polyvinyl alcohol is not particularly limited, and may be appropriately selected according to the intended purpose. Examples of the polyvinyl alcohol include, but are not limited to, silanol-modified polyvinyl alcohol, carboxyl-modified polyvinyl alcohol, and acetoacetyl-modified polyvinyl alcohol.

The above-listed polyvinyl alcohols may be used alone or in combination.

### --Polyvinyl acetate--

The polyvinyl acetate is not particularly limited, and may be appropriately selected according to the intended purpose. Examples of the polyvinyl acetate include, but are not limited to, a vinyl acetate-crotonic acid copolymer and a vinyl acetate-itaconic acid copolymer.

The above-listed polyvinyl acetates may be used alone or in combination.

### --Biodegradable polyester--

The biodegradable polyester is not particularly limited, and may be appropriately selected according to the intended purpose. Examples of the biodegradable polyester include, but are not limited to, polylactic acid, poly-ε-caprolactone, succinate-based polymers (e.g., polyethylene succinate, polybutylene succinate, and polybutylene succinate adipate), polyhydroxy alkanoate (e.g., polyhydroxy propionate, polyhydroxy butyrate, and polyhydroxy valerate, and polyglycolic acid. Among the above-listed biodegradable polyesters, polylactic acid is preferable because polylactic acid has high biocompatibility, and allows sustained release of the bioactive substance.

The above-listed biodegradable polyesters may be used alone or in combination.

A weight average molecular weight of the polylactic acid is not particularly limited, and may be appropriately selected according to the intended purpose. The weight average molecular weight of the polylactic acid is preferably 5,000 or greater and 100,000 or less, more preferably 10,000 or greater and 70,000 or less, yet more preferably 10,000 or greater and 50,000 or less, and particularly preferably 10,000 or greater and 30,000 or less.

An amount of the polylactic acid is not particularly limited, and may be appropriately selected according to the intended purpose. The amount of the polylactic acid is preferably 50 percent by mass or greater, more preferably 50 percent by mass or greater and 99 percent by mass or less, yet more preferably 75 percent by mass or greater and 99 percent by mass or less, and particularly preferably 80 percent by mass or greater and 99 percent by mass or less, relative to a total mass of the base material.

### --Polyglycolic acid--

The polyglycolic acid is not particularly limited, and may be appropriately selected according to the intended purpose. Examples of the polyglycolic acid include, but are not limited to: a lactic acid-glycolic acid copolymer that is a copolymer including a constituent unit derived from lactic acid and a constituent unit derived from glycolic acid; a glycolic acid-caprolactone copolymer that is a copolymer including a constituent unit derived from glycolic acid and a constituent unit derived from caprolactone; and a glycolic acid-trimethylene carbonate copolymer that is a copolymer including a constituent unit derived from glycolic acid and a constituent unit derived from trimethylene carbonate. Among the above-listed polyglycolic acids, the lactic acid-glycolic acid copolymer is preferable because the lactic acid-glycolic acid copolymer has high biocompatibility, allows sustained release of the bioactive substance, and can preserve the bioactive substance over a long period.

The above-listed polyglycolic acids may be used alone or in combination.

A weight average molecular weight of the lactic acid-glycolic acid copolymer is not particularly limited, and may be appropriately selected according to the intended purpose. The weight average molecular weight of the lactic acid-glycolic acid copolymer is preferably 2,000 or greater and 250,000 or less, more preferably 2,000 or greater and 100,000 or less, yet more preferably 3,000 or greater and 50,000 or less, and particularly preferably 5,000 or greater and 10,000 or less.

A molar ratio (L:G) of a constituent unit (L) derived from lactic acid to a constituent unit (G) derived from glycolic acid in the lactic acid-glycolic acid copolymer is not particularly limited, and may be appropriately selected according to the intended purpose. The molar ratio (L:G) is preferably from 1:99 to 99:1, more preferably from 25:75 to 99:1, yet more preferably from 30:70 to 90:10, and particularly preferably from 50:50 to 85:15.

An amount of the lactic acid-glycolic acid copolymer is not particularly limited, and may be appropriately selected according to the intended purpose. The amount of the lactic acid-glycolic acid copolymer is preferably 50 percent by mass or greater, more preferably 50 percent by mass or greater and 99 percent by mass or less, yet more preferably 75 percent by mass or greater and 99 percent by mass or less, and particularly preferably 80 percent by mass or greater and 99 percent by mass or less, relative to a total amount of the base material.

### --Polyamino acid--

The polyamino acid is not particularly limited, and may be appropriately selected according to the intended purpose. Examples of the polyamino acid include, but are not limited to, polymers of valine, lysine, leucine, threonine, isoleucine, asparagine, glutamine, phenylalanine, aspartic acid, serine, glutamic acid, methionine, arginine, glycine, alanine, tyrosine, proline, histidine, cysteine, tryptophan, derivatives of the foregoing, and combinations of the foregoing.

The polyamino acid is preferably a polymer of a single amino acid. Examples of the preferable polyamino acid include, but are not limited to, homopolymers or copolymers of an amino acid, such as poly-α-glutamic acid, poly-γ-glutamic acid, polyaspartic acid, polylysine, polyarginine, polyornithine, and polyserine.

The above-listed polyamino acids may be used alone or in combination.

### --Gelatin--

The gelatin is not particularly limited, and may be appropriately selected according to the intended purpose. Examples of the gelatin include, but are not limited to, lime-processed gelatin, acid processed-gelatin, gelatin hydrolysate, enzymatically dispersed gelatin, and the derivatives of the foregoing.

The above-listed gelatins may be used alone or in combination.

The gelatin derivative refers to a gelatin that is derived by covalently binding a hydrophobic group to a gelatin molecule. The hydrophobic group is not particularly limited, and may be appropriately selected according to the intended purpose. Examples of the hydrophobic group include, but are not limited to: polyesters, such as polylactic acid, polyglycolic acid, and poly-ε-caprolactone; lipids, such as cholesterol and phosphatidyl ethanolamine; alkyl groups; aromatic groups including benzene rings; heterocyclic aromatic groups; and a mixture of the foregoing.

A natural dispersant polymer used for the gelatin derivative is not particularly limited, and may be appropriately selected according to the intended purpose. Examples of the natural dispersant polymer include, but are not limited to, proteins, polysaccharides, and nucleic acids. Examples of the natural dispersant polymer also include a copolymer including the natural dispersant polymer or a synthetic dispersant polymer.

The above-listed natural dispersant polymers may be used alone or in combination.

The proteins used as the natural dispersant polymer are not particularly limited, as long as the proteins do not adversely affect the biological activity of the bioactive substance. The proteins may be appropriately selected according to the intended purpose. Examples of the proteins include, but are not limited to, collagen, fibrin, and albumin.

The above-listed proteins may be used alone or in combination.

The polysaccharides used as the natural dispersant polymer are not particularly limited, and may be appropriately selected according to the intended purpose. Examples of the polysaccharides include, but are not limited to, chitin, chitosan, hyaluronic acid, alginic acid, starches, and pectin.

The above-listed polysaccharides may be used alone or in combination.

The particles obtained by the method for producing the particles of the present disclosure may include two or more base materials. In the case where one of the two or more base materials is included to be locally present at the surface side of each particle, the base materials are not particularly limited, and may be appropriately selected according to the intended purpose, but at least one base material is preferably a base material having pH responsiveness. More preferably, the base material that is contained to be locally distributed at the surface side of each particle is the base material having pH responsiveness.

The pH responsiveness refers to a characteristic that solubility of the base material changes in response to pH. For example, enteric particles can be produced by incorporating the base material having pH responsiveness, which is dissolved at pH 5.0 or higher, in a manner such that the base material having pH responsiveness is arranged to be locally distributed at the surface side of each particle.

The base material having pH responsiveness is not particularly limited, and may be appropriately selected according to the intended purpose. Examples of the base material having pH responsiveness include cellulose-based polymers, methacrylic acid-based polymers, vinyl-based polymers, amino acids, chitosan, pectin, and alginic acid. Among the above-listed base materials, at least one selected from the group consisting of the cellulose-based polymers and the methacrylic acid-based polymers is preferable because the base material is easily locally distributed at the surface side of each particle during the production of the particles, and the enteric solubility can be improved.

The above-listed base materials may be used alone or in combination.

Examples of the cellulose-based polymers include, but are not limited to, hydroxypropyl methyl cellulose acetate succinate, hydroxypropyl methyl cellulose phthalate, carboxymethyl ethyl cellulose, and cellulose acetate trimellitate. Among the above-listed cellulose-based polymers, at least one selected from the group consisting of hydroxypropyl methyl cellulose acetate succinate and hydroxypropyl methyl cellulose phthalate is preferable because the cellulose-based polymer is easily locally arranged at the surface sides of the particles during the production of the particles, and the enteric solubility can be improved.

The above-listed cellulose-based polymers may be used alone or in combination.

Examples of the methacrylic acid-based polymers include, but are not limited to, aminoalkyl methacrylate copolymers, methacrylic acid copolymers, methacrylate copolymers, and ammonioalkyl methacrylate copolymers. Among the above-listed methacrylic acid-based polymers, the ammonioalkyl methacrylate copolymer is preferable because the ammonioalkyl methacrylate copolymer is easily locally arranged at the surface sides of the particles during the production of the particles, and the enteric solubility can be improved.

The above-listed methacrylic acid-based polymers may be used alone or in combination.

A combination of the base materials is not particularly limited, and may be appropriately selected according to the intended purpose. In the case where two base materials are used, for example, a combination of at least one selected from the group consisting of poly(meth)acrylic acid, polyglycolic acid, and hydroxypropyl methyl cellulose, and at least one selected from the group consisting of hydroxypropyl cellulose, polyethylene pyrrolidone, and polyalkylene glycol is preferable.

In view of biocompatibility, the base material is preferably a substance that is not toxic to a living body, and more preferably a biodegradable substance, such as a biodegradable polymer.

An amount of the base material is preferably 5 percent by mass or greater and 95 percent by mass or less, and more preferably 50 percent by mass or greater and 95 percent by mass or less, relative to a total mass of the particles.

When the amount of the base material is 5 percent by mass or greater and 95 percent by mass or less relative to a total mass of the particles, redispersibility of the bioactive substance is improved. Thus, the amount of the base material in the above range is preferable.

### <<<Solvent>>>

The solvent is not particularly limited, and may be appropriately selected according to the intended purpose. Examples of the solvent include, but are not limited to, water, aliphatic halogenated hydrocarbons (e.g., dichloromethane, dichloroethane, and chloroform), alcohols (e.g., methanol, ethanol, and propanol), ketones (e.g., acetone and methyl ethyl ketone), ethers (e.g., diethyl ether, dibutyl ether, and 1,4-dioxane), aliphatic hydrocarbons (e.g., n-hexane, cyclohexane, and n-heptane), aromatic hydrocarbons (e.g., benzene, toluene, and xylene), organic acids (e.g., acetic acid and propionic acid), esters (e.g., ethyl acetate), amides (e.g., dimethylformamide and dimethylacetamide), and solvent mixtures of the foregoing. Among the above-listed solvents, aliphatic halogenated hydrocarbons, alcohols, or solvent mixtures of the foregoing are preferable, and dichloromethane, 1,4-dioxane, methanol, ethanol, and solvent mixtures of the foregoing are more preferable, in view of solubility.

The above-listed solvents may be used alone or in combination.

An amount of the solvent is preferably 70 percent by mass or greater and 99.8 percent by mass or less, and more preferably 90 percent by mass or greater and 99 percent by mass or less, relative to a total amount of the bioactive-substance-containing liquid.

When the amount of the solvent is 70 percent by mass or greater and 99.8 percent by mass or less relative to the total amount of the bioactive-substance-containing liquid, production stability is improved in terms of solubility of the bioactive substance and the viscosity of the bioactive-substance-containing liquid.

### <<<Other ingredients>>>

Other ingredients are not particularly limited, as long as the other ingredients do not adversely affect the biological activity of the bioactive substance. Other ingredients may be appropriately selected according to the intended purpose. Examples of the other ingredients include, but are not limited to, surfactants. In the case where the particles are included in a pharmaceutical composition, a functional food, or functional cosmetics, a bioactive-substance-containing liquid is preferably substantially free from other ingredients for improving safety.

In the present specification, "substantially free" means equal to or lower than a detection limit.

A form of the bioactive-substance-containing liquid is not particularly limited, and may be appropriately selected according to the intended purpose. For example, the bioactive-substance-containing liquid may be a water-in-oil (W/O) emulsion.

When the form of the bioactive-substance-containing liquid is a W/O emulsion, drying can be performed at a low temperature as an organic solvent having a low boiling point is used. Even in the case where the bioactive substance whose biological activity is easily changed by heating is included, a change in the biological activity of the bioactive substance can be minimized. As a result, a reduction in the biological activity level of the bioactive substance can be minimized.

A production method of the W/O emulsion is not particularly limited, and may be appropriately selected according to the intended purpose. For example, a liquid A in which the bioactive substance is dissolved in a solvent A and a liquid B (solvent B) are mixed and stirred, thereby preparing a W/O emulsion in which the liquid A is dispersed in the liquid B. The W/O emulsion may be a W/O emulsion that includes air bubbles trapped during stirring, or a W/O emulsion from which air bubbles are removed.

In the W/O emulsion prepared in the above manner, the liquid A is present as droplets in the liquid B. In addition, the bioactive substance is present in the droplets of the liquid A. Since the bioactive substance is present in the droplets of the liquid A, the bioactive substance can be protected, and a change in the biological activity of the bioactive substance by stirring can be minimized. As a result, a reduction in the biological activity level of the bioactive substance can be minimized.

The liquid A and the liquid B are solvents that are immiscible with each other. The solvent A and the liquid B (solvent B) are more preferably water and dichloromethane, respectively.

A method for producing the W/O emulsion may include a dispersion step in which a dispersion liquid is prepared.

The dispersion step is not particularly limited, and may be appropriately selected according to the intended purpose. Examples of the dispersion step include, but are not limited to, a method in which the liquid A and the liquid B are mixed and stirred. Specific examples of the stirring method include, but are not limited to, stirring using a stirrer or a homogenizer.

In the present specification, the "dispersion liquid" refers to a suspension liquid in which one substance (dispersoid, liquid A) is suspended as fine particles in another substance (dispersion medium, liquid B).

### -Solvent A-

The solvent A is not particularly limited, as long as the solvent A can dissolve the bioactive substance. The solvent A may be appropriately selected according to the intended purpose. Examples of the solvent A include, but are not limited to, water and hexafluoro-2-propanol (HFIP).

An amount of the solvent A is not particularly limited, and may be appropriately selected according to the intended purpose. The amount of the solvent A is preferably 50 percent by mass or greater and 99.9 percent by mass or less, and more preferably 90 percent by mass or greater and 99.9 percent by mass or less, relative to a total amount of the liquid A.

When the amount of the solvent A is 50 percent by mass or greater and 99.9 percent by mass or less relative to the total amount of the liquid A, a stable dispersion liquid can be produced.

### -Solvent B-

The solvent B is not particularly limited, and may be appropriately selected according to the intended purpose. Examples of the solvent B include, but are not limited to, dichloromethane, methyl acetate, and hexafluoro-2-propanol.

An amount of the solvent B is not particularly limited, and may be appropriately selected according to the intended purpose. The amount of the solvent B is preferably 50 percent by mass or greater and 99.9 percent by mass or less, and more preferably 70 percent by mass or greater and 99.9 percent by mass or less, relative to a total amount of the liquid B.

When the amount of the solvent B is 50 percent by mass or greater and 99.9 percent by mass or less relative to the total amount of the liquid B, a stable dispersion liquid can be produced.

As a quantity ratio between the liquid A and the liquid B in the dispersion liquid, a ratio (liquid A mass : liquid B mass) of a mass of the liquid A to a mass of the liquid B is preferably 0.1:99.9 to 40:70, and more preferably 0.5:99.5 to 30:80.

A droplet size (diameter) of the liquid A in the dispersion liquid is not particularly limited, and may be appropriately selected according to the intended purpose. The droplet size of the liquid A is preferably 50 nm or greater and 10 µm or less, and more preferably 100 nm or greater and 5 µm or less.

When the droplet size of the liquid A in the dispersion liquid is 50 nm or greater, discharge defects, which are caused by coalescence of droplets, can be inhibited during discharge of droplets.

When the droplet size of the liquid A in the dispersion liquid is 10 µm or less, discharge defects can be inhibited during discharge of droplets.

An amount of the bioactive substance in the dispersion liquid is not particularly limited, and may be appropriately selected according to the intended purpose. The amount of the bioactive substance is preferably 0.05 percent by mass or greater and 50 percent by mass or less, and more preferably 0.1 percent by mass or greater and 20 percent by mass or less, relative to a total amount of the liquid A.

Additives other than the bioactive substance may be dissolved in the liquid A and in the liquid B.

The additives are not particularly limited, and may be appropriately selected according to the intended purpose. Examples of the additives include, but are not limited to, a surfactant and a buffer solution. Among the above-listed additives, a surfactant is preferably added to the liquid B in view of stability of a W/O emulsion.

The surfactant is not particularly limited, and may be appropriately selected according to the intended purpose. Examples of the surfactant include, but are not limited to, benzalkonium chloride, lecithin, sorbitan fatty acid esters, polyoxyethylene hydrogenated castor oil, polyoxyethylene polyoxypropylene glycol, polyoxyethylene sorbitan monooleate, polyvinyl alcohol, sorbitan monooleate, glycerin monostearate, sorbitan sesquioleate, sorbitan monostearate, glyceryl monostearate, polyglycerol fatty acid ester, polysorbate (e.g., polysorbate 80), polyoxyethylene-polyoxypropylene copolymers, and sodium lauryl sulfate.

The above-listed surfactants may be used alone or in combination.

A hydrophilic-lipophilic balance (HLB) of the surfactant, which is an index of the surfactant, is not particularly limited, and may be appropriately selected according to the intended purpose. The HLB value of the surfactant is preferably 1 or greater and 8 or less, and more preferably 3 or greater and 6 or less. Among the above-listed surfactants, sorbitan sesquioleate (HLB 4) is particularly preferable.

An amount of the surfactant is not particularly limited, and may be appropriately selected according to the intended purpose. The amount of the surfactant is preferably 0.05 percent by mass or greater and 5 percent by mass or less, and more preferably 0.05 percent by mass or greater and 1 percent by mass or less, relative to a total amount of the liquid B.

When the amount of the surfactant is 0.05 percent by mass or greater and 5 percent by mass or less, formed particles can be suitably collected as a powder. In other words, when the amount of the surfactant is less than 0.05 percent by mass, the base material is not sufficiently dispersed, and when the amount of the surfactant is greater than 5 percent by mass, the interface tension of the base material is weak so that the formed particles cannot be collected as a powder.

A viscosity of the bioactive-substance-containing liquid is not particularly limited, and may be appropriately selected according to the intended purpose. The viscosity of the bioactive-substance-containing liquid is preferably 0.5 mPa·s or greater and 15.0 mPa·s or less, and more preferably 0.5 mPa·s or greater and 10.0 mPa·s or less.

When the viscosity of the bioactive-substance-containing liquid is 0.5 mPa·s or greater and 15.0 mPa·s or less, droplets of the bioactive-substance-containing liquid can be desirably discharged.

The viscosity of the bioactive-substance-containing liquid can be measured, for example, by a viscoelasticity measurement device (device name: MCR rheometer, manufactured by AntonPaar) at 25°C and at the shear rate of 10 s⁻¹.

A surface tension of the bioactive-substance-containing liquid is not particularly limited, and may be appropriately selected according to the intended purpose. The surface tension of the bioactive-substance-containing liquid is preferably 10 mN/m or greater and 60 mN/m or less, and more preferably 20 mN/m or greater and 50 mN/m or less.

The surface tension of the bioactive-substance-containing liquid can be measured, for example, by a portable surface tensiometer (device name: PocketDyne, manufactured by KRUSS) at 25°C with a lifetime of 1,000 ms according to the maximum foaming pressure method.

### <Bioactive-substance-containing liquid storage container>

The bioactive-substance-containing liquid storage container is a container in which the bioactive-substance-containing liquid is accommodated.

A material of the bioactive-substance-containing liquid storage container is not particularly limited, and may be appropriately selected according to the intended purpose. The material may be flexible or not flexible, and may be formed of a resin or a metal.

A structure of the bioactive-substance-containing liquid storage container is not particularly limited, and may be appropriately selected according to the intended purpose. For example, the structure of the bioactive-substance-containing liquid storage container may be a sealed structure or an unsealed structure.

### <Discharging step and discharger>

The discharging step includes discharging the bioactive-substance-containing liquid as droplets.

The discharger is a device configured to discharge the bioactive-substance-containing liquid as droplets.

The discharger is coupled to the bioactive-substance-containing liquid storage container, for example, by piping (e.g. a pipe or a tube).

### <<Discharging step of first embodiment>>

The discharging step of the first embodiment includes discharging the bioactive-substance-containing liquid into a gas.

### <<Discharging step of second embodiment>>

The discharging step of the second embodiment includes discharging the bioactive-substance-containing liquid into a vacuum.

### <<Discharging step of third embodiment>>

The discharging step of the third embodiment includes discharging the bioactive-substance-containing liquid into a liquefied gas.

The common features of the discharging step of the first to thirds embodiments will be described hereinafter.

A method for discharging droplets is not particularly limited, and may be appropriately selected according to the intended purpose. Examples of the discharging method are as follows:
(i) a method using a discharger, in which the pressurized bioactive-substance-containing liquid is discharged as droplets from one or more holes formed in a flat plate nozzle surface, such as an inkjet nozzle;
(ii) a method using a discharger in which the pressurized bioactive-substance-containing liquid is discharged as droplets from one or more holes having irregular shapes, such as an SPG membrane; and
(iii) a method using a discharger in which the bioactive-substance-containing liquid is discharged from one or more discharging holes by vibration.

Among the above-listed methods, (iii) is preferable.

In the case where the discharging method (iii) is used as the method for discharging droplets, the discharger preferably includes a vibration applying member configured to apply vibration to the bioactive-substance-containing liquid, thereby discharging droplets of the bioactive-substance-containing liquid.

The vibration applying member is not particularly limited, and may be appropriately selected according to the intended purpose. Examples of the vibration applying member include, but are not limited to, an inkjet nozzle.

In the discharging method (iii), examples of a discharge mechanism of the inkjet nozzle include devices in which external stress is hardly applied to the bioactive-substance-containing liquid, such as a discharger using a membrane vibration method, a discharger using Rayleigh breakup, a discharger using a liquid vibration method, and a discharger using liquid column resonance. The above discharging methods can minimize a change in the biological activity of the bioactive substance due to vibration.

The above dischargers may include a device configured to apply pressure to the bioactive-substance-containing liquid, thereby discharging the bioactive-substance-containing liquid.

Among the above-listed discharging methods, a discharging method using a device, which is a discharger using the liquid column resonance and is configured to apply pressure to the bioactive-substance-containing liquid to discharge the bioactive-substance-containing liquid, is preferable.

Examples of the discharger using the liquid column resonance include, but are not limited to, a discharger using a method in which vibration is applied to the bioactive-substance-containing liquid accommodated in the liquid column resonance liquid storage chamber to generate standing waves, and the bioactive-substance-containing liquid is discharged from one or more discharges hole formed in regions corresponding to antinodes of the standing waves in the amplitude direction of the standing waves.

Examples of the discharger using the membrane vibration method include, but are not limited to, the discharger disclosed in Japanese Unexamined Patent Application Publication No. 2008-292976.

Examples of the discharger using the Rayleigh breakup include, but are not limited to, the discharger disclosed in Japanese Patent No. 4647506.

Examples of the discharger using the liquid vibration method include, but are not limited to, the discharger disclosed in Japanese Unexamined Patent Application Publication No. 2010-102195.

As one example of the discharging step, a method for discharging the bioactive-substance-containing liquid as droplets by vibration will be newly described.

The method for discharging the bioactive-substance-containing liquid by vibration is not particularly limited. Examples of the discharging method include, but are not limited to, the following methods:
(a) a method using a volume changing device that is configured to change a volume of a liquid container using vibration;
(b) a method using a constriction generating device in which the bioactive-substance-containing liquid is discharged from one or more discharge holes formed in a liquid container, while applying vibration to the liquid container, so that the columnar shapes of the bioactive-substance-containing liquid are constricted to thereby be formed into droplets; and
(c) a method using a nozzle vibration unit configured to vibrate a thin film in which nozzles are formed.

The volume changing device is not particularly limited, as long as the volume changing device can change the volume of the liquid container. The volume changing device may be appropriately selected according to the intended purpose. Examples of the volume changing device include, but are not limited to, a piezoelectric element that expands and contracts when a voltage is applied.

Examples of the constriction generating device include, but are not limited to, devices using the technology disclosed in Japanese Unexamined Patent Application Publication No. 2007-199463. The device for forming droplets is disclosed in Japanese Unexamined Patent Application Publication No. 2007-199463. In the disclosed device, the liquid is discharged from nozzle holes formed in a liquid container, while applying vibration to the liquid container by a vibration device using a piezoelectric element in contact with a part of the liquid container, so that the columnar shapes of the liquid are constricted to be formed into droplets.

Examples of the vibration device include, but are not limited to, devices using the technology disclosed in Japanese Unexamined Patent Application Publication No. 2008-292976. The device for forming droplets is disclosed in Japanese Unexamined Patent Application Publication No. 2008-292976. The disclosed device is configured to discharge the liquid from nozzle holes to form into droplets using a thin film, in which the nozzle holes are formed, and a piezoelectric element. The thin film is provided to the liquid container. The piezoelectric element is disposed in the periphery of the deformable region of the thin film, and vibrates the thin film.

The piezoelectric element is not particularly limited, and a shape, size, and material of the piezoelectric element are appropriately selected. For example, a piezoelectric element used for an inkjet discharging system in the related art can be used.

The shape and size of the piezoelectric element are not particularly limited, and may be appropriately selected according to shapes of the discharge holes or the like.

The material of the piezoelectric element is not particularly limited, and may be appropriately selected according to the intended purpose. Examples of the material of the piezoelectric element include, but are not limited to: piezoelectric ceramics, such as lead zirconate titanate (PZT); piezoelectric polymers, such as poly vinylidene fluoride (PVDF); and single crystals, such as quartz, LiNbO₃, LiTaO₃, and KNbO₃.

The one or more discharge holes are not particularly limited, and may be appropriately selected according to the intended purpose. Examples of the one or more discharge holes include, but are not limited to, openings formed in a nozzle plate.

A cross-sectional shape of the discharge hole is not particularly limited, and may be appropriately selected according to the intended purpose. Examples of the cross-sectional shape of the discharge hole include, but are not limited to: (1) a tapered shape in which an opening diameter decreases from the inner side (liquid container side) to the outer side (side to which the bioactive-substance-containing liquid is discharged); (2) a shape in which an opening diameter decreases from the inner side (liquid container side) to the outer side (side to which the bioactive-substance-containing liquid is discharged), while maintaining a round shape; (3) a shape in which an opening diameter decreases from the inner side (liquid container side) to the outer side (side to which the bioactive-substance-containing liquid is discharged) at a constant nozzle angle; and (4) a combination of the shape of (1) and the shape of (2).

Among the above-listed shapes, the shape of (3) is preferable because the maximum pressure is applied to the bioactive-substance-containing liquid at the discharge hole.

The nozzle angle in the shape of (3) is not particularly limited, and may be appropriately selected according to the intended purpose. The nozzle angle is preferably 60° or greater and 90° or less in view of stabilized discharge of the bioactive-substance-containing liquid.

A size of the discharge hole is not particularly limited, and may be appropriately selected according to the intended purpose. The diameter of the discharge hole is preferably less than 1,000 µm, more preferably 1.0 µm or greater and less than 1,000 µm, yet more preferably 1.0 µm or greater and 500 µm or less, and particularly preferably 1.0 µm or greater and 50 µm or less. In the case where a shape of the discharge hole is not a perfect circle, a diameter of a perfect circle having an area equivalent to an area of the discharge hole is determined as the diameter of the discharge hole.

The vibration is not particularly limited, and may be appropriately selected according to the intended purpose. A frequency of the vibration is preferably 1 kHz or greater, more preferably 150 kHz or greater, and yet more preferably 300 kHz or greater and 500 kHz or less.

When the frequency is 1 kHz or greater, the liquid column discharged from the discharge hole can be formed into droplets with high reproducibility. When the frequency is 150 kHz or greater, production efficiency can be improved.

### <Particle formation step and particle forming device>

The particle formation step includes removing a solvent from the droplets to form particles.

The particle forming device is a device configured to remove a solvent from the droplets to form particles.

In the present specification, "removing" or "remove" means removal of the solvent included in the liquid phase, but is not limited to a case where the entire solvent included in the liquid phase is removed. Part of the solvent included in the liquid phase may remain as long as particles can be formed.

A method for removing the solvent is not particularly limited, and may be appropriately selected according to the intended purpose. Examples of the method include, but are not limited to: a method in which a liquid phase is brought into contact with a gas to diffuse a solvent included in the liquid phase into another gas phase; and a method in which a solvent included in a liquid phase is vaporized in a vacuum or a liquefied gas to freeze the liquid phase in the above process, followed by performing freeze-drying.

### <<Particle formation step of first embodiment>>

The particle formation step of the first embodiment is performed in a gas. Specifically, the particle formation step is preferably performed when the droplets discharged into the gas in the discharging step are flying in the gas. By performing the particle formation step, the obtained particles are formed in the form of dispersed solids, specifically, the form in which the bioactive substance is dispersed in the base material.

Various methods are known as a dry particle formation method for forming particles in a gas, as in the particle formation step of the first embodiment. Examples of the dry particle formation method include, but are not limited to: a method in which a melt-kneaded product obtained by melting and kneading constituent materials of particles to homogeneously disperse the constituent materials, followed by cooling, is pulverized by a pulverizer to obtain pulverized particles having a small particle size; air pulverization, such as a method in which a liquid including constituent materials of particles is freeze-dried, followed by pulverizing the freeze-dried product by a pulverizer to obtain pulverized particles having a small particle size; and a spray dry method (spray drying) in which a liquid including constituent materials of particles is sprayed and dried in a gas to thereby obtain atomized particles having a small particle size.

Examples of the method for spraying the liquid into the gas include, but are not limited to: a pressure nozzle method in which a liquid is pressurized and discharged from a nozzle; and a disk method in which a liquid is sent to a disk rotating at a high speed, thereby scattering the liquid by a centrifugal force.

The air pulverization uses simple equipment for pulverization. However, it is difficult to produce particles having a narrow particle size distribution in the air pulverization.

In the case where the bioactive substance whose biological activity is likely to be changed by heating is included as a constituent material of particles in the method for pulverizing the melt-kneaded product, the biological activity of the bioactive substance may be changed, which lowers the biological activity level of the bioactive substance in the particles.

In the case where the bioactive substance whose biological activity is likely to be changed by cooling is included as a constituent material of particles in the method for pulverizing the freeze-dried product, the biological activity of the bioactive substance may be changed, which lowers the biological activity level of the bioactive substance in the particles.

In the air pulverization, large external stress is generated during the process of pulverization. In the case where the biological activity is likely to be changed by external stress is included as a constituent material of particles, the biological activity of the bioactive substance may be changed, which lowers the biological activity level of the bioactive substance in the particles.

In the spray drying, particles having a large amount of the bioactive substance remaining in the particles (high bioactive substance retention ratio) can be produced. However, it is difficult to produce particles having a small particle size in the spray drying. When the method for spraying the liquid into the gas is the disk method in the spray drying, particles having a small particle size may be produced, but large-scale equipment needs to be used.

In the spray drying, droplets are likely to coalesce in the air, and it is therefore difficult to produce particles having a narrow particle size distribution. In order to inhibit coalescence of droplets in the air, it is important to heat the sprayed droplets so that the droplets dry immediately after being sprayed. In the case where the bioactive substance whose biological activity is likely to be changed by heating is included as a constituent material of particles, the biological activity of the bioactive substance may be changed, which lowers the biological activity level of the bioactive substance in the particles.

In the spray drying, large external stress is generated by spraying, as a high shear force is applied to spray. In the case where the bioactive substance whose biological activity is likely to be changed by external stress is included as a constituent material of particles, the biological activity of the bioactive substance may be changed, which lowers the biological activity level of the bioactive substance in the particles.

The method for producing the particles of the present disclosure does not correspond to the above air pulverization nor the above spray drying. The method for producing the particles of the present disclosure does not perform heating to dry the droplets, or does not use external stress to reduce diameters of the droplets. Therefore, even in the case where the bioactive substance whose biological activity is likely to be changed by heating or external stress is included as a constituent material of particles, a change in the biological activity of the bioactive substance can be minimized, and as a result, a reduction in the biological activity level of the bioactive substance can be minimized during the production of the particles.

In the method for producing the particles of the present disclosure, droplets having the substantially uniform size are discharged and formed into particles, while controlling the droplets to inhibit coalescence. Therefore, particles having a uniform size can be stably produced. Specifically, the particles having a narrow particle size distribution can be obtained.

In the particle formation step of the first embodiment, the bioactive substance does not come into contact with a solvent, such as water, during the formation of the particles, and therefore the amount of the bioactive substance remaining in the particles (bioactive substance retention ratio) can be increased. The bioactive substance retention ratio in percentage is, for example, 80% or greater.

In the particle formation step of the first embodiment, droplets may be discharged into a conveying gas stream, and the solvent is vaporized and removed from the droplets, thereby forming particles. In this case, the particle forming device of the first embodiment may include a conveying gas stream generator that generates a conveying gas stream.

A method for vaporizing the solvent from the droplets using the conveying gas stream is not particularly limited, and may be appropriately selected according to the intended purpose. Examples of the method include, but are not limited to: a method in which a conveying direction of the conveying gas stream is set to a substantially perpendicular direction to the direction in which the droplets are discharged; and a method in which a conveying direction of the conveying gas stream is set to the same direction as the direction in which the droplets are discharged.

A temperature, vapor pressure, and gas of the conveying gas stream are preferably appropriately adjusted. In the case where heating is performed to adjust the temperature of the conveying gas stream, the temperature is preferably a temperature at which the biological activity of the bioactive substance does not change.

As long as the particles collected after the particle formation step of the first embodiment are in the solid state, the solvent may not be completely vaporized. In this case, a drying step, or a step of vaporizing the solvent from the droplets using a temperature change or chemical change may be separately provided as a post-step.

In the case where the bioactive-substance-containing liquid includes two or more base materials and particles in which one of the base materials is locally distributed at the surface side of each particle are formed, the contact angles of the two or more base materials are preferably different from one another. In such a configuration, the interaction between the base materials increases, and therefore a phase separation of the base materials likely occurs when the solvent is vaporized in the particle formation step, thereby forming particles in which one of the two or more base materials is locally distributed to the surface side in each particle.

A difference between the contact angles of the two or more base materials is not particularly limited, and may be appropriately selected according to the intended purpose. The difference between the contact angles of the two or more base materials is preferably 1.0° or greater, and more preferably 10.0° or greater, in view of facilitation of phase separation.

A method for measuring the contact angle of the base material is not particularly limited, and may be appropriately selected according to the intended purpose. Examples of the method include, but are not limited to, a method for measuring a contact angle using a contact angle meter. Examples of the contact angle meter include, but are not limited to, a pocket goniometer PG-X+/portable contact angle meter, manufactured by FIBRO system.

A method for confirming the presence or absence of the phase separation between two or more base materials is not particularly limited, and may be appropriately selected according to the intended purpose. Examples of the method include, but are not limited to, a method in which a solution obtained by dissolving two or more base materials in a good solvent is applied into a thin film by a bar coater, and observing the state of the thin film before, during, and after drying under an optical microscope (e.g., OLYMPUS BX51, manufactured by Olympus Corporation).

In the case where the solvent used together with the base materials is lipophilic, as one base material that is included to be locally distributed at the surface side of each particle, a base material having a larger contact angle than the contact angle of another base material included to be locally distributed at the inner side in each particle is selected. Since the one base material having a large contact angle has a large affinity to the solvent than another base material included to be distributed at the inner side of each particle, the one base material is likely to be included to be locally distributed toward the solvent side, i.e., the surface side of each particle.

In the case where particles including a water-soluble bioactive substance are desired to be produced, a lipophilic solvent is used so that resultant particles have a configuration in which the bioactive substance is included to be locally distributed at the inner side of each particle, and the inner side of each particle is coated with one base material that is included to be locally distributed at the surface side of each particle.

In the case where the solvent used together with the base materials is hydrophilic, as one base material that is included to be locally distributed at the surface side of each particle, a base material having a smaller contact angle than the contact angle of another base material included to be locally distributed at the inner side in each particle is selected. Since the one base material having a small contact angle has a smaller affinity to the solvent than another base material included to be distributed at the inner side of each particle, the one base material is likely to be included to be locally distributed toward the solvent side, i.e., the surface side of each particle.

In the case where particles including a lipid-soluble bioactive substance are desired to be produced, a hydrophilic solvent is used so that resultant particles have a configuration in which the bioactive substance is included to be locally distributed at the inner side of each particle, and the inner side of each particle is coated with one base material that is included to be locally distributed at the surface side of each particle.

As the one base material that is included to be locally distributed at the surface side of each particle, a material having pH responsiveness is preferably used. In the case where enteric particles to be included in a pharmaceutical composition are produced, at least one selected from the group consisting of cellulose-based polymers and methacrylic acid-based polymers is used as the material having pH responsiveness in view of larger contact angles of the above materials than another base material.

Among the cellulose-based polymers, at least one selected from the group consisting of hydroxypropyl methyl cellulose acetate succinate and hydroxypropyl methyl cellulose phthalate is preferable in view of larger contact angles of the above cellulose-based polymers than another base material.

Among the acrylic acid-based polymers, an ammonioalkyl methacrylate copolymer is preferable in view of a larger contact angle of the ammonioalkyl methacrylate copolymer than another base material.

A combination of one base material included to be locally distributed at the surface side of each particle and another base material included to be locally distributed at the inner side of each particle is not particularly limited, and may be appropriately selected according to the intended purpose. In view of phase separation without mutual compatibility, a combination of one selected from the group consisting of poly(meth)acrylic acid, polyglycolic acid, and hydroxypropyl methyl cellulose, and one selected from the group consisting of hydroxypropyl cellulose, polyethylene pyrrolidone, and polyalkylene glycol is preferable.

### <<Particle formation step of second embodiment>>

The particle formation step of the second embodiment is performed in a vacuum. Specifically, a preferable embodiment is such that the solvent is vaporized from the droplets discharged into the vacuum in the discharging step and the liquid phase is frozen in the above process, followed by performing freeze-drying to remove the solvent.

In the second embodiment, the description overlapping with the first embodiment will be omitted.

The effect obtained by the particle formation step of the second embodiment is the same as the effect obtained by the particle formation step of the first embodiment. In the particle formation step of the second embodiment, droplets are immediately freeze-dried after discharging, different from spray drying of the related art. Thus, in addition to the effect obtained by the particle formation step of the first embodiment, even in the case where the bioactive substance whose biological activity is likely to be changed particularly by heating, a change in the biological activity of the bioactive substance can be minimized, and as a result, a reduction in the biological activity level of the bioactive substance can be minimized.

The particle forming device of the second embodiment may include a freeze-drying device. The freeze-drying device is not particularly limited, and may be appropriately selected according to the intended purpose.

### <<Particle formation step of third embodiment>>

The particle formation step of the third embodiment is performed in a liquefied gas. Specifically, a preferable embodiment is such that the droplets discharged into the liquefied gas in the discharging step are cooled, and the liquid phase is frozen in the above process, followed by performing freeze-drying to remove the solvent.

In the third embodiment, the description overlapping with the first and second embodiments will be omitted.

The effect obtained by the particle formation step of the third embodiment is the same as the effect obtained by the particle formation step of the second embodiment.

The particle forming device of the third embodiment is the same as the particle forming device of the second embodiment.

In the method for producing the particles of the present disclosure, a biological activity ratio {(biological activity level B/biological activity level A)×100} of the biological activity level B of the bioactive substance after the bioactive-substance-containing liquid preparation step to the biological activity level A of the bioactive substance before the bioactive-substance-containing liquid preparation step is 80% or greater.

The biological activity ratio affects the biological activity retention ratio or the bioactive substance retention ratio.

The biological activity retention ratio is a retention ratio of the biological activity of the bioactive substance in the particles after the production of the particles. The biological activity of the bioactive substance may be changed by heating or external stress. If processing involving heating, shaking, or stirring is performed in the production process of the particles, the biological activity retention ratio is reduced, and as a result, the biological activity ratio is also reduced.

The bioactive substance retention ratio refers to a ratio of the bioactive substance remaining in the particles. For example, the bioactive substance is lost due to decomposition or elution in the production process of the particles, and as a result, a total amount of the bioactive substance retained in the particles may be reduced. In this case, the bioactive substance retention ratio is reduced, and as a result, the biological activity ratio is also reduced.

A method for establishing the method for producing the particles, which achieves the biological activity ratio of 80% or greater, is not particularly limited, and may be appropriately selected according to the intended purpose. Examples of the method include, but are not limited to, a method in which application of heating and external stress is not performed on the bioactive-substance-containing liquid and droplets of the bioactive-substance-containing liquid.

### <Other steps and other devices>

Other steps are not particularly limited, and may be appropriately selected according to the intended purpose. Examples of the other steps include, but are not limited to, a particle collecting step.

Other devices are not particularly limited, and may be appropriately selected according to the intended purpose. Examples of the other devices include, but are not limited to, a particle collector.

The particle collecting step includes collecting produced particles.

The particle collector is not particularly limited, and may be appropriately selected according to the intended purpose. Examples of the particle collector include, but are not limited to, a cyclone collector and a back filter.

A particle production apparatus in association with the present disclosure will be described with reference to drawings, hereinafter. However, the present disclosure is not limited to the following embodiments in any way.

In the drawings, the same constituent components are denoted by the same reference numerals, and redundant description may be omitted. The number, position, or shape of each constituent component is not limited to those in the present embodiment, and may be any number, position, or shape preferable for implementing the present disclosure.

### [Fig. 1]

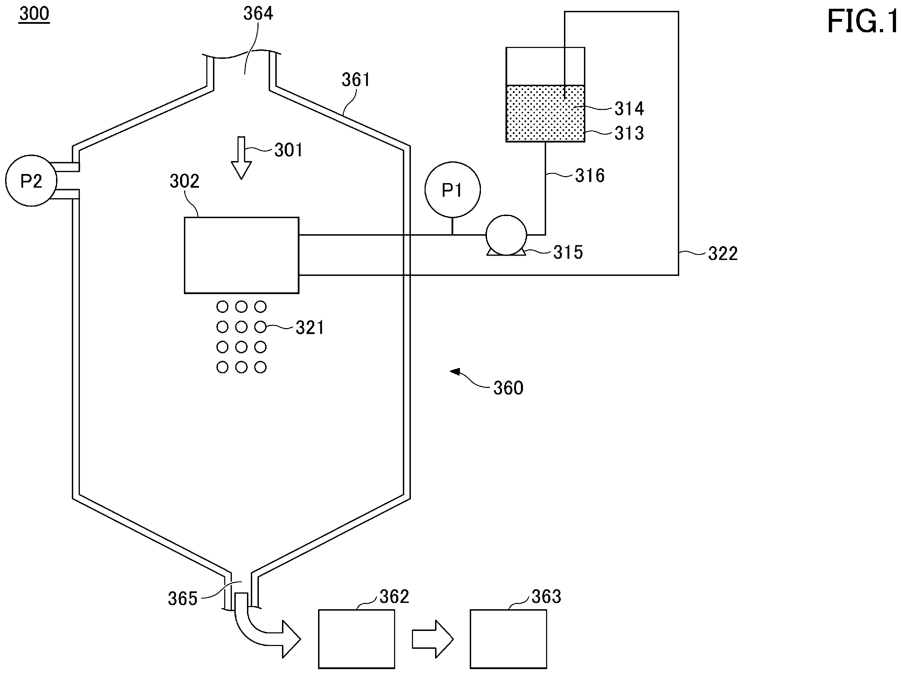

Fig. 1 is a schematic view illustrating one example of the particle production apparatus of the first embodiment of the present disclosure.

As illustrated in Fig. 1, the particle production apparatus 300 includes a discharger 302, a drying and collecting device 360, a conveying gas stream outlet 365, and a particle storage 363.

A bioactive-substance-containing liquid storage container 313 that accommodates the bioactive-substance-containing liquid 314, and a liquid circulation pump 315 that pressure-feeds the bioactive-substance-containing liquid 314 in a liquid supply pipe 316 are coupled to the discharger 302.

The liquid circulation pump 315 supplies the bioactive-substance-containing liquid 314 accommodated in the bioactive-substance-containing liquid storage container 313 to the discharger 302 through the liquid supply pipe 316, and sends the bioactive-substance-containing liquid 314 back to the bioactive-substance-containing liquid storage container 313 through a liquid return pipe 322. Thus, the bioactive-substance-containing liquid 314 is supplied to the discharger 302 as needed.

A pressure gauge P1 is provided to the liquid supply pipe 316. A pressure gauge P2 is provided to the drying and collecting device 360. The pressure of the liquid fed to the discharger 302 and the pressure inside the drying and collecting device 360 are monitored by the pressure gauge P1 and the pressure gauge P2, respectively.

The above pressures are preferably controlled so that the pressure value measured by the pressure gauge P1 and the pressure value measured by the pressure gauge P2 are substantially the same. In the case where the pressure value measured by the pressure gauge P1 is greater than the pressure value measured by the pressure gauge P2, the bioactive-substance-containing liquid 314 may ooze out from the discharge holes. In the case where the pressure value measured by the pressure gauge P1 is smaller than the pressure value measured by pressure gauge P2, a gas may enter the discharger 302, and stop the discharge.

A conveying gas stream 301 (downward gas stream) flowing from a conveying gas stream inlet 364 is formed in a chamber 361. Droplets 321 discharged from the discharger 302 are transported downward by the gravity and the conveying gas stream 301, pass through the conveying gas stream outlet 365, are collected by a particle collector 362, and then are stored in the particle storage 363.

In the discharging step, if discharged droplets come into contact with one another before drying, the droplets may coalesce with one another. Specifically, the discharged droplets travel at a constant initial velocity, but slow down due to air resistance. Then, the traveling droplets may be caught up by the droplets discharged later. In this case, if the droplets are not sufficiently dried, droplets may coalesce with one another.

Therefore, the droplets are preferably transported by the conveying gas stream while drying the droplets with the conveying gas stream in view of inhibition of reduction in the traveling speed of the droplets, and inhibition of contact between the droplets.

The conveying gas stream 301 is arranged in the same direction as the discharge direction of the droplets in the vicinity of the discharger 302.

### [Fig. 2]

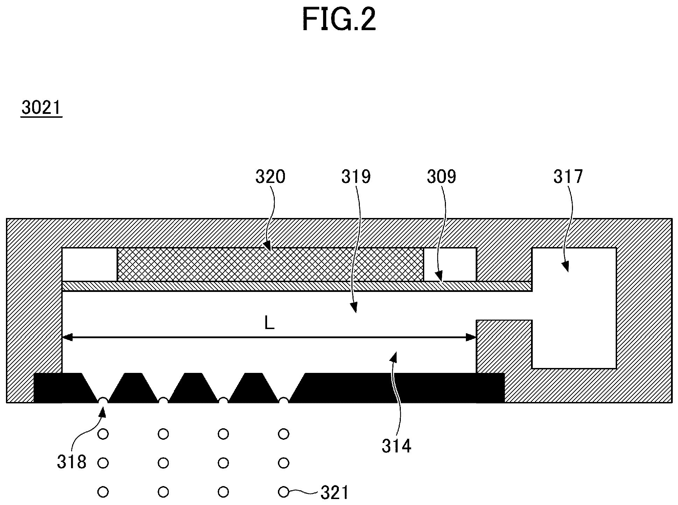

Fig. 2 is a schematic cross-sectional view illustrating one example of a liquid-column-resonance droplet discharger 3021 serving as the discharger 302 in the particle production apparatus 300 of the first embodiment of the present disclosure.

The liquid-column-resonance droplet discharger 3021 includes a common liquid supply path 317 and a liquid-column-resonance liquid chamber 319.

The liquid-column-resonance liquid chamber 319 communicates with the common liquid supply path 317 provided on one of the wall surfaces at both ends in the longitudinal direction.

The liquid-column-resonance liquid chamber 319 includes discharge holes 318 and a vibration generator 320. The discharge holes 318 are formed in one of wall surfaces connected to wall surfaces of both ends, and discharge droplets 321. The vibration generator 320 is disposed on a wall surface facing the discharge holes 318, and generates high frequency vibration to form liquid column resonance standing waves. A high frequency powder source is coupled to the vibration generator 320.

A gas stream passage for supplying a gas stream that transports the discharged droplets 321 may be provided to the liquid-column-resonance droplet discharger 3021.

The bioactive-substance-containing liquid 314 is fed into the common liquid supply path 317 of the liquid-column-resonance droplet discharger 3021 through the liquid supply pipe by the liquid circulation pump, and is then supplied to the liquid-column-resonance liquid chamber 319.

In the liquid-column-resonance liquid chamber 319 filled with the bioactive-substance-containing liquid 314, a pressure gradient is formed by the liquid column resonance standing waves generated by the vibration generator 320. The droplets 321 are discharged from the discharge holes 318 disposed in the regions (regions where the amplitude is large and the pressure fluctuation is large) corresponding to the antinodes of the liquid column resonance standing waves.

The regions corresponding to the antinodes of the liquid column resonance standing waves are regions other than regions corresponding to nodes of the standing waves, preferably regions where the pressure fluctuations due to the standing waves have the amplitude large enough to discharge the bioactive-substance-containing liquid 314, and more preferably regions corresponding to the ±1/4 wavelength of a region from the position where the amplitude of the pressure standing wave becomes the maximum (node as the velocity standing wave) to the position where the amplitude becomes the minimum.

As long as the discharge holes are disposed in the regions corresponding to the antinodes of the standing waves, even when multiple discharge holes are provided, substantially uniform droplets can be formed, and droplets can be efficiently discharged. In addition, clogging of the discharge holes are less likely to occur.

The bioactive-substance-containing liquid 314 that has passed through the common liquid supply path 317 is circulated through the liquid return pipe. When the amount of the bioactive-substance-containing liquid 314 in the liquid-column-resonance liquid storage chamber 319 is reduced by discharging the droplets 321, a suction force due to the action of the liquid column resonance standing waves in the liquid-column-resonance liquid chamber 319 is exhibited, and a flow rate of the bioactive-substance-containing liquid 314 supplied from the common liquid supply path 317 is increased. Then, the liquid-column-resonance liquid chamber 319 is replenished with the bioactive-substance-containing liquid 314.

The liquid-column-resonance liquid chamber 319 of the liquid-column-resonance droplet discharger 3021 is composed of a frame that is formed of a material having a high rigidity. The level of the rigidity of the material is a rigidity that does not affect the resonance frequency of the liquid at the drive frequency. Examples of the material having the high rigidity include, but are not limited to, metals, ceramics, and silicone.

As illustrated in Fig. 2, the length L between the wall surfaces of the liquid-column-resonance liquid chamber 319 at the both ends in the longitudinal direction is determined based on the liquid column resonance principle.

In order to significantly improve productivity, a plurality of liquid-column-resonance liquid chambers 319 are disposed relative to one droplet forming device.

The number of the liquid-column-resonance liquid chambers 319 is not particularly limited, and may be appropriately selected according to the intended purpose. The number of the liquid-column-resonance liquid chambers 319 is preferably 1 or greater and 2,000 or less.

The common liquid supply path 317 may have a configuration such that a flow path for supplying the liquid is coupled to each liquid-column-resonance liquid chamber 319 to communicate with the common liquid supply path 317, and the common liquid supply path 317 communicates with a plurality of liquid-column-resonance liquid chambers 319.

The vibration generator 320 of the liquid-column-resonance droplet discharger 3021 is not particularly limited, as long as the vibration generator 320 can be driven at a predetermined frequency. The vibration generator 320 may be appropriately selected according to the intended purpose. A preferable embodiment of the vibration generator 320 is such that the vibration generator 320 is bonded to an elastic plate 309 constituting part of the wall of the liquid-column-resonance liquid chamber so that a piezoelectric body does not come into contact with the liquid.

In view of productivity, the frequency is preferably 150 kHz or greater, and more preferably 300 kHz or greater and 500 kHz or less.

Examples of the piezoelectric body include, but are not limited to, piezoelectric ceramics, such as lead zirconate titanate (PZT). Since an amount of displacement of the piezoelectric body is small, the piezoelectric body is often used by stacking layers of the piezoelectric body. Other examples of the piezoelectric body include: piezoelectric polymers, such as polyvinylidene fluoride (PVDF); and single crystals, such as quartz, LiNbO₃, LiTaO₃, and KNbO₃.

The vibration generator 320 is preferably provided to each of the liquid-column-resonance liquid chambers 319 so that the liquid-column-resonance liquid chambers 319 are individually controlled.

As the discharge holes 318, a plurality of openings may be provided for improving productivity. In this case, the discharge holes 318 are aligned in the width direction of the liquid-column-resonance liquid chamber 319. The frequency of the liquid column resonance changes depending on the arrangement of the openings of the discharge holes 318, and therefore the liquid column resonance frequency is desirably appropriately determined by checking the discharge of the droplets.

In the case where the amount of the residual solvent in the particles obtained by the particle collector 362 is large, secondary drying is preferably performed. For secondary drying, a generally known drying method, such as fluidized bed drying or vacuum drying, can be carried out.

### [Fig. 3]

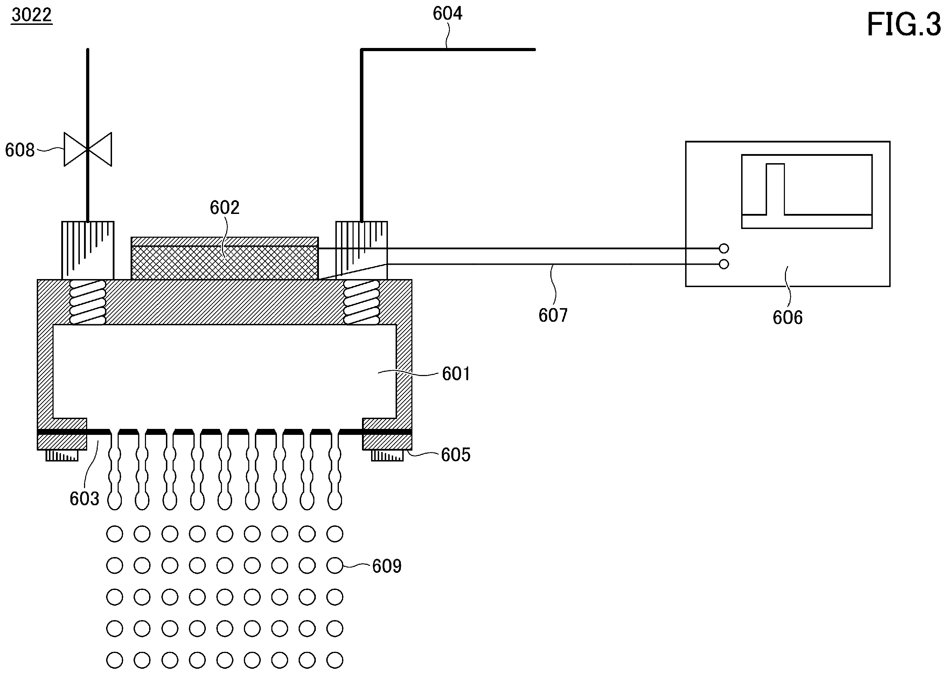

The particle production apparatus 300 may employ a discharger 3022 using Rayleigh breakup instead of the above-described liquid-column-resonance droplet discharger 3021. Fig. 3 is a schematic cross-sectional view illustrating one example of the discharger 3022 using Rayleigh breakup serving as the discharger 302 of the particle production apparatus 300 of the first embodiment of the present disclosure.

The discharger 3022 using Rayleigh breakup includes a reservoir 601 in which the bioactive-substance-containing liquid is accommodated, a vibrator 602, and a plurality of through holes 603.

A material of the reservoir 601 is not particularly limited, and may be appropriately selected according to the intended purpose. The material is preferably a material that has resistance to the pressure of approximately 10 MPa because the bioactive-substance-containing liquid is accommodated in the pressurized state. Specific examples of the material include metals, such as SUS and aluminum.

The reservoir 601 is coupled to a supply pipe 604 for supplying the bioactive-substance-containing liquid to the reservoir 601.

To the reservoir 601, a through-hole plate holding mechanism 605 that holds a plate having through holes 603 for discharging droplets 609 is provided.

The vibrator 602 that vibrates the entire reservoir 601 is provided to the reservoir 601 in a manner such that the vibrator 602 is in contact with the reservoir 601. A vibration generator 606 and a conductive wire 607 are coupled to the vibrator 602.

The vibrator 602 is preferably a vibrator that can oscillate the entire reservoir 601 using one vibration generator. The vibrator 602 is not particularly limited, as long as the vibrator 602 can apply vibration at a constant frequency, and may be appropriately selected according to the intended purpose. For example, the vibrator 602 may be configured to apply vibration at a constant frequency by expansion and contraction of a piezoelectric body.

The constant frequency is not particularly limited, and may be appropriately selected according to the intended purpose. The constant frequency is preferably 10 kHz or greater and 10 MHz or less. In view of generation of fine droplets that realize an extremely uniform particle size, the constant frequency is more preferably 50 kHz or greater and 2 MHz or less.

The reservoir 601 is provided with an open valve 608 for adjusting the pressure or removing air bubbles inside in view of stable formation of liquid columns.

### [Fig. 4]

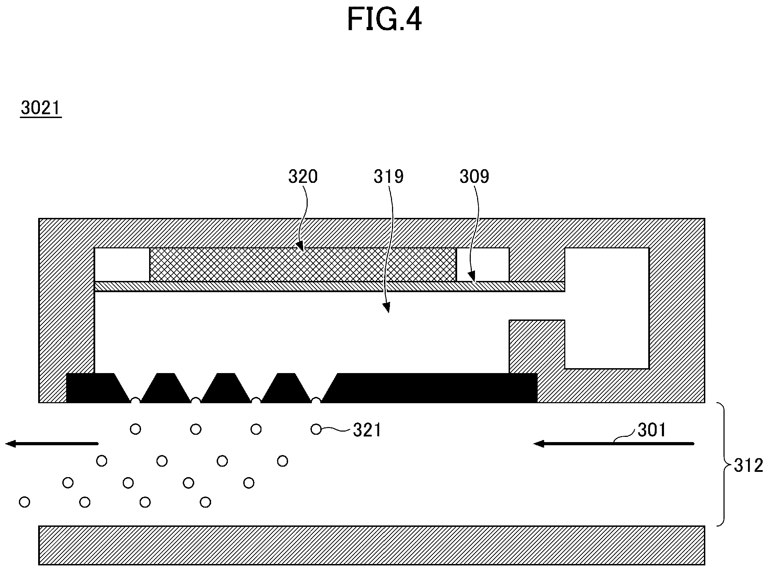

Fig. 4 is a schematic view illustrating another example of the particle production apparatus of the first embodiment of the present disclosure. The description of the same configuration as that of the particle production apparatus of the first embodiment will be omitted.

As illustrated in Fig. 4, the conveying gas stream 301 may be arranged in a substantially perpendicular direction to the discharge direction in the gas stream passage 312.

In view of inhibition of coalescence of droplets with one another, the conveying gas stream 301 may be at an angle as long as the conveying gas stream 301 is in direction along which the droplets 321 move away from the discharger 302. The positions of the discharge holes 318 are preferably adjusted so that trajectories of the droplets 321 transported by the conveying gas stream 301 do not overlap.

After inhibiting coalescence of the droplets 321 using the conveying gas stream 301, the resultant particles may be transported to the particle collector using another gas stream.

The speed of the conveying gas stream 301 is preferably equal to or faster than the discharge speed. When the speed of the conveying gas stream is faster than the discharge speed, coalescence of droplets with one another can be inhibited.

A chemical substance that facilitates drying of the droplets 321 may be mixed into the conveying gas stream 301.

A state of the conveying gas stream 301 is not particularly limited, and may be appropriately selected according to the intended purpose. For example, the conveying gas stream 301 may be a laminar flow, a swirling flow, or a turbulent flow.

A gas constituting the conveying gas stream 301 is not particularly limited, and may be appropriately selected according to the intended purpose. Examples of the gas include, but are not limited to, inflammable gasses, such as air and nitrogen.

### [Fig. 5]

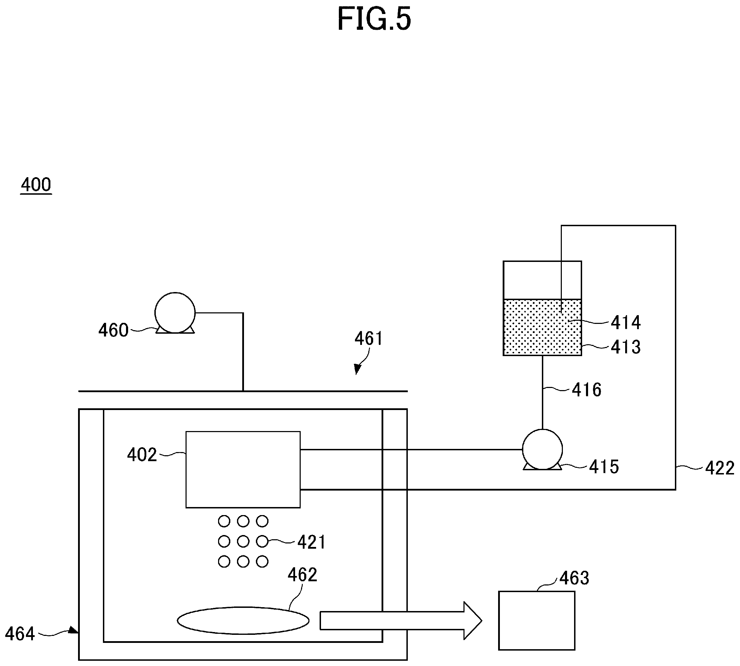

Fig. 5 is a schematic view illustrating an example of the particle production apparatus 400 of a second embodiment of the present invention.

The particle production apparatus 400 illustrated in Fig. 5 includes a discharger 402, a chamber 461, a vacuum pump 460, and a freeze-drying device 463.

A bioactive-substance-containing liquid storage container 413, in which the bioactive-substance-containing liquid 414 is accommodated, and a liquid circulation pump 415 that pressure-feeds the bioactive-substance-containing liquid 414 in a liquid supply pipe 416 are coupled to the discharger 402.

The liquid circulation pump 415 supplies the bioactive-substance-containing liquid 414, which is accommodated in the bioactive-substance-containing liquid storage container 413, to the discharger 402 through the liquid supply pipe 416, and returns the bioactive-substance-containing liquid 414 back to the bioactive-substance-containing liquid storage container 413 through the liquid return pipe 422. Thus, the bioactive-substance-containing liquid 414 is supplied to the discharger 402 as needed.

The chamber 461 is a sealed container and includes a cooling jacket through which a refrigerant 464 flows.

The chamber 461 is evacuated by a vacuum pump 460 to maintain a vacuum.

The solvent in the droplets 421 discharged in the chamber 461 is evaporated, and the droplets 421 are frozen by the latent heat of evaporation to thereby form a frozen product 462.

In the discharging step, if the discharged droplets 421 come into contact with one another before being frozen, the droplets 421 may coalesce with one another. In view of formation of particles having a narrow particle size distribution, a sufficient distance between the discharged droplets 421 is preferably maintained. In view of inhibition of coalescence of the droplets 421 with one another, a conveying gas stream may be arranged, and the droplets 421 may be frozen while being transported.

The frozen product 462 is taken out from the particle production apparatus 400, and the obtained frozen product 462 is freeze-dried by the freeze-drying device 463, to thereby obtain dried particles. The freeze-drying device is not particularly limited, and may be appropriately selected according to the intended purpose. For example, the freeze-drying device may be a batch-type freeze-drying device or a continuous freeze-drying device. Among the above devices, the continuous freeze-drying device is preferable in view of productivity.

### [Fig. 6]

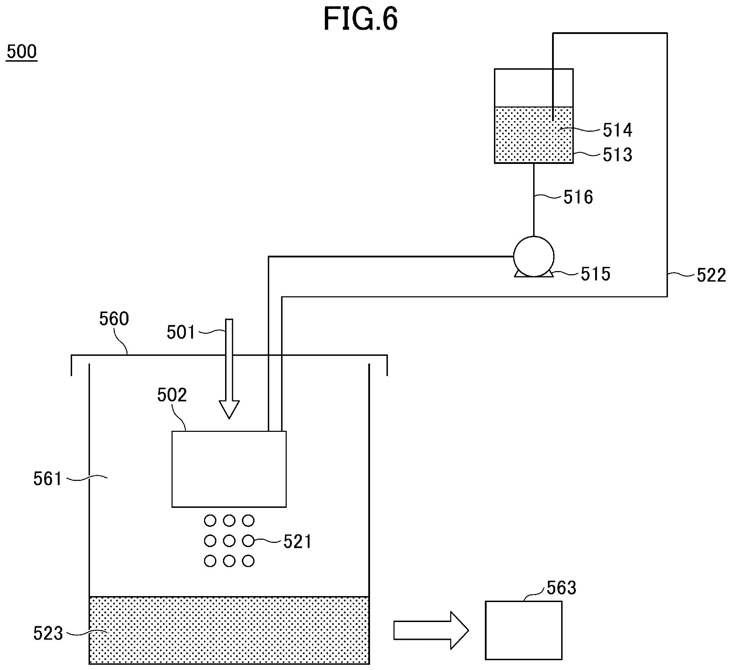

Fig. 6 is a schematic view illustrating an example of the particle production apparatus 500 of the third embodiment of the present disclosure.

As illustrated in Fig. 6, the particle production apparatus 500 includes a discharger 502, a chamber lid 560, a chamber 561, and a freeze-drying device 563.

A bioactive-substance-containing liquid storage container 513, in which the bioactive-substance-containing liquid 514 is accommodated, and a liquid circulation pump 515 that pressure-feeds the bioactive-substance-containing liquid 514 in a liquid supply pipe 516 are coupled to the discharger 502.

The liquid circulation pump 515 supplies the bioactive-substance-containing liquid 514, which is accommodated in the bioactive-substance-containing liquid storage container 513, to the discharger 502 via the liquid supply pipe 516, and returns the bioactive-substance-containing liquid 514 back to the bioactive-substance-containing liquid storage container 513 through the liquid return pipe 522. Thus, the bioactive-substance-containing liquid 514 is supplied to the discharger 502, as needed.

The chamber 561 is a heat resistant container, and preferably has a Dewar vessel structure. The chamber 561 is filled with a liquefied gas 523.

The liquefied gas 523 is preferably externally stirred by a stirring device, such as a magnetic stirrer.

The chamber lid 560 is preferably an open-type container because the gas evaporated from the liquefied gas 523 flows out.

In the chamber 561, the droplets 521 discharged from the discharger 502 are transported downward by gravity and the conveying gas stream 501, and the droplets 521 are cooled by the liquefied gas 523 to thereby freeze the droplets 521. The frozen droplets are dispersed in the liquefied gas 523.

In the discharging step, if the discharged droplets come into contact with one another before being frozen, the droplets may coalesce with one another. Specifically, the discharged droplets travel at a constant initial speed, but slow down due to air resistance. Thus, the droplets discharged later may reach the traveling droplets that have been previously discharged. In this case, if the droplets are not sufficiently dried, the droplets may coalesce with one another.

Therefore, the droplets are preferably transported by the conveying gas stream 501 while drying the droplets in view of inhibition of a reduction in the traveling speed of the droplets.

The conveying gas stream 501 is preferably arranged in the same direction as the discharge direction of the droplets 521 in the vicinity of the discharger 502.

The frozen droplets 521 dispersed in the liquefied gas 523 are taken out from the particle production apparatus 500 while being in the chamber 561, and the frozen droplets 521 are freeze-dried by the freeze-drying device 563 to thereby obtain dried particles.

### (Particles)

The particles of the present disclosure are particles produced by the above-described method for producing the particles of the present disclosure.

In the present specification, the term "particle" refers to a droplet of the bioactive-substance-containing liquid or a semi-dried or dried droplet of the bioactive-substance-containing liquid, unless otherwise stated.

The particles of the present disclosure are preferably functional particles that exhibit a desired function. The functional particles can be designed to exhibit a desired function by appropriately selecting a base material to be included in the bioactive-substance-containing liquid.

Examples of the functional particles include, but are not limited to: particles that deliver a bioactive substance to a target site to exhibit a desired physiological effect, i.e., particles used for a drug delivery system (DDS); sustained-release particles that release a drug over a long period of time; solubilizing particles for solubilizing a sparingly soluble bioactive substance; immediate-release particles; pH-dependent release particles; pH-independent release particles; enteric coated particles; controlled-release coated particles; and nanocrystal-containing particles.

Examples of the form of the particles used for the drug delivery system (DDS) include, but are not limited to: particles in which the bioactive substance itself is formed into particles; capsule particles in which the bioactive substance is encapsulated in the base material; carrier particles in which the bioactive substance is borne on surfaces of particles of the base material, and particles in other forms.

Examples of the capsule particles include, but are not limited to, dispersed capsule particles in which the bioactive substance is dispersed and encapsulated in the base material, and locally distributed capsule particles in which the bioactive substance is locally distributed and encapsulated in the base material.

The state of being "encapsulated" in the capsule particles is not particularly limited, as long as the bioactive substance is not temporarily or continuously retained in the base material.

The dispersed capsule particles are not particularly limited as long as the bioactive substance is dispersed and encapsulated in the base material. The degree of dispersion of the bioactive substance in the base material may not be uniform. **In** the case where the particles include a plurality of base materials, and one of the base materials is locally distributed to a predetermined position inside each particle, the degree of the dispersion may be different depending on the base material present in the area where the bioactive substance is encapsulated.

Examples of the particles corresponding to the dispersed capsule particles include, but are not limited to, particles produced by emulsion solvent diffusion (ESD), and particles produced by spray drying.

The locally distributed capsule particles are in the form of particles in which the bioactive substance is locally distributed and encapsulated in the base material, in other words, in the form of particles in which the base material in each particle and the bioactive substance are substantially separately located to encapsulate the bioactive substance in the base material. Examples of the form of the locally distributed capsule particles include, but are not limited to, a form of particles in which each particle includes a center portion including the bioactive substance, and a peripheral portion including the base material and encapsulating the center portion.

Examples of particles corresponding to the locally distributed capsule particles include, but are not limited to, liposomes, micelles, and coated particles.

The carrier particles are in the form of particles in which the bioactive substance is borne on the surface of the base material by adsorption or bonding. Examples of the adsorption include, but are not limited to, chemisorption and physisorption. Examples of the bonding include, but are not limited to, hydrogen bonding, covalent bonding, ionic bonding, and chelate bonding.

Examples of the particles corresponding to the carrier particles include, but are not limited to, porous particles in which the bioactive substance is borne on a surface (not only an external surface but also an internal surface) of the porous base material.

The particles of the present disclosure may include two or more base materials. In the case where the particles include two or more base materials, one of the base materials may be locally distributed at the surface side of each particle. In this case, the bioactive substance is dispersed and encapsulated in both the base material locally distributed at the surface side of each particle (may be referred to as a "surface base material" hereinafter) and a base material other than the surface base material (may be referred to as an "inner base material" hereinafter).

Specific examples of the present embodiment include, but are not limited to: an embodiment in which the bioactive substance is locally distributed and included in the surface material; and an embodiment in which the bioactive substance is locally distributed and included in the inner base material. Among the above embodiments, the embodiment in which the bioactive substance is locally distributed and included in the inner base material is preferable. Since the bioactive substance is locally distributed and included in the inner base material, sustained-release particles in which the elution speed of the bioactive substance is regulated can be obtained.

A method for confirming that the particles are in the form, in which the particles include two or more base materials, and one of the two or more base materials is locally distributed at the surface side of each particle, is not particularly limited, and may be appropriately selected according to the intended purpose. Examples of the method include, but are not limited to, a method in which cross-sections of the particles are observed under a scanning electron microscope, a transmission electron microscope, or a scanning probe microscope.

Another example of the method is a method in which a component of the surface base material is measured using time-of-flight secondary ion mass spectrometry to determine whether the surface base material is different from the component of the inner base material, thereby confirming the above form of the particles.

As another method for confirming the form of the particles, a pre-treatment, such as electron staining or a dissolution treatment, can be performed. **In** the case of the particles including the base material composed of a water-soluble substance and the base material composed of a water-insoluble substance, for example, cross-sections of the particles, which have been immersed in water to completely dissolve the water-soluble ingredient, may be observed under a scanning electron microscope, thereby determining that the water-insoluble ingredient is distributed in the remaining portion of the cross-sections of the particles, and the water-soluble ingredient is distributed to the void portions of the cross-sections of the particles.

A relative span factor (R.S.F.) of the particles is 1.5 or less in view of drying of the particles at a low temperature and inhibition of reduction in the biological activity ratio. The R.S.F is an index representing the breadth of the particle size distribution of the particles, and is determined by (D90 - D10)/D50. D90 is a value at 90 percent by volume of a cumulative particle size distribution from the side of the small particle side. D50 is a value at 50 percent by volume of a cumulative particle size distribution from the side of the small particle side. D10 is a value at 10 percent by volume of a cumulative particle size distribution from the side of the small particle side.

When the value of the R.S.F. of the particles is large, the particle size distribution is broadened, and a large amount of a coarse powder is contained. In order to dry the coarse powder, a drying temperature is set high, and therefore the biological activity ratio may be reduced. Therefore, the R.S.F. is preferably 1.2 or less, more preferably 1.0 or less, and yet more preferably 0.6 or less.

A method for measuring the R.S.F. is not particularly limited, and may be appropriately selected according to the intended purpose. Examples of the method include, but are not limited to, a method for measuring by a concentration system analyzer ("FPAR-1000" manufactured by Otsuka Electronics Co., Ltd.) according to dynamic light scattering.

The volume average particle diameter (Dv) of the particles is 1 µm or greater and 50 µm or less. When the volume average particle diameter (Dv) of the particles is 1 µm or greater and 50 µm or less, a sufficient amount of the bioactive substance can be retained within the particles so that, for example, sustained-release particles that can gradually release the bioactive substance over a long period can be produced.

A method for measuring the volume average particle diameter (Dv) of the particles is not particularly limited, and may be appropriately selected according to the intended purpose. Examples of the method include, but are not limited to, a method for measuring using a concentration system analyzer ("FPAR-1000" manufactured by Otsuka Electronics Co., Ltd.) according to dynamic light scattering; and a method for measuring using a laser diffraction-scattering particle size distribution analyzer (device name: Microtrac MT3000II, manufactured by MicrotracBEL Corp.).

A ratio Dv/Dn of the volume average particle diameter (Dv) of the particles to the number average particle diameter (Dn) of the particles is not particularly limited, and may be appropriately selected according to the intended purpose. In view of drying of the particles at a low temperature and minimization of reduction in the biological activity ratio, the ratio Dv/Dn is preferably 1.00 or greater and 2.0 or less, and more preferably 1.00 or greater and 1.50 or less.

A method for measuring the number average particle diameter (Dn) is not particularly limited, and may be appropriately selected according to the intended purpose. Examples of the method include, but are not limited to, a method for measuring Dn using a laser diffraction-scattering particle size distribution analyzer (device name: Microtrac MT3000II, manufactured by MicrotracBEL Corp.).

The particle size distribution of the particles is not particularly limited, and may be appropriately selected according to the intended purpose. For example, it is preferable that R.S.F. is 1.2 or less, and the ratio Dv/Dn of the volume average particle diameter to the number average particle diameter is 1.00 or greater and 1.50 or less.

When the particle size distribution of the particles is within the above ranges, a ratio of particles that correspond to coarse particles can be reduced. Thus, even when it is necessary to use a pharmaceutical composition including the particle after filtration sterilization, filtration sterilization can be performed simply and efficiently without clogging a filter of the filtration sterilization.

Since the size of the particles becomes homogeneous, the amounts of the bioactive substance and the base material in each particle, and a surface area of each particle become uniform. Thus, an amount of the bioactive substance eluted from each of the particles becomes uniform, and the particles in which sustained-release of the bioactive substance is controlled at a high level can be obtained.

Since the size of the particles becomes homogeneous, generation of small-size particles, which are composed of particles of the bioactive substance alone without the base material, can be minimized, and the sustained-release particles that inhibit initial burst can be obtained.

### [Use]

The particles obtained by the method for producing the particles of the present disclosure can be used, for example, as a pharmaceutical composition, a functional food, or functional cosmetics by combining other ingredients, such as a dispersant and additives, as necessary.

### <Pharmaceutical composition>

The pharmaceutical composition of the present disclosure includes the particles obtained by the above-described method for producing the particles, and lipids, and may further include other ingredients as necessary.

The particles in the pharmaceutical composition are the same as the above-described particles, and therefore redundant description will be omitted.

### <<Lipids>>

The lipids are not particularly limited, and may be appropriately selected according to the intended purpose. Examples of the lipids include, but are not limited to, medium or long chain monoglyceride, medium or long chain diglyceride, medium or long chain triglyceride, phospholipid, vegetable oil (e.g., soybean oil, avocado oil, squalene oil, sesame oil, olive oil, corn oil, rapeseed oil, safflower oil, and sunflower oil), fish oil, seasoning oil, water-insoluble vitamins, fatty acids, medium-chain triglyceride, mixtures of the foregoing, and derivatives of the foregoing.

The above-listed lipids may be used alone or in combination.

In the case where the particles including the bioactive substance are dispersed in a lipid to prepare a pharmaceutical composition as an injection preparation, it is desired that a low force is used for discharging the composition from a syringe. As an amount of the particles in the pharmaceutical composition increases, therefore, a viscosity of the pharmaceutical composition increases. Therefore, the amount of the particles dispersed in the lipid is preferably 1 mg/mL or greater and 500 mg/mL or less.

The ratio µ1/µ0 of a viscosity µ1 to a viscosity µ0 is preferably 10 or less, where the viscosity µ1 is the viscosity of the pharmaceutical composition when the amount of the particles in the pharmaceutical composition is 400 mg/mL, and the viscosity µ0 is the viscosity of the pharmaceutical composition when the amount of the particles in the pharmaceutical composition is 0 mg/mL.

The above configuration is preferable because the force for discharging the composition from the syringe can be reduced in the case where the pharmaceutical composition is an injection preparation.

Other ingredients are not particularly limited, and may be appropriately selected according to the intended purpose. Examples of other ingredients include, but are not limited to, excipients, flavoring agents, disintegrating agents, fluidizers, adsorbents, lubricants, odor-masking agents, surfactants, fragrances, colorants, antioxidants, masking agents, anti-static agents, and humectants.

The above-listed ingredients may be used alone or in combination.

### -Excipients-

The excipients are not particularly limited, and may be appropriately selected according to the intended purpose. Examples of the excipients include, but are not limited to, lactose, sucrose, mannitol, glucose, fructose, maltose, erythritol, maltitol, xylitol, palatinose, trehalose, sorbitol, crystalline cellulose, talc, silicic acid anhydride, anhydrous calcium phosphate, precipitated calcium carbonate, and calcium silicate.

The above-listed excipients may be used alone or in combination.

### -Flavoring agents-

The flavoring agents are not particularly limited, and may be appropriately selected according to the intended purpose. Examples of the flavoring agents include, but are not limited to, L-menthol, refined sugar, D-sorbitol, xylitol, citric acid, ascorbic acid, tartaric acid, malic acid, aspartame, acesulfame potassium, thaumatin, saccharin sodium, dipotassium glycyrrhizinate, sodium glutamate, sodium 5'-inosinate, and sodium 5'-guanylate.

The above-listed flavoring agents may be used alone or in combination.

### -Fluidizers-

The fluidizers are not particularly limited, and may be appropriately selected according to the intended purpose. Examples of the fluidizers include, but are not limited to, light anhydrous silicic acid, hydrated silicon dioxide, and talc.

The above-listed fluidizers may be used alone or in combination.

As light anhydrous silicic acid, a commercial product may be used. The commercial product of the light anhydrous silicic acid is not particularly limited, and may be appropriately selected according to the intended purpose. Examples of the commercial product include, but are not limited to, ADSOLIDER 101 (manufactured by Freund Corporation, average pore diameter: 21 nm).

### -Adsorbents-

As the adsorbents, commercial products may be used. The commercial products of the adsorbents are not particularly limited, and may be appropriately selected according to the intended purpose. Examples of the commercial product include, but are not limited to, product name: CARPLEX (component name: synthetic silica, registered trademark of DSL. Japan Co., Ltd.), product name: AEROSIL (registered trademark of NIPPON AEROSIL CO., LTD.) 200 (component name: hydrophilic fumed silica), product name: SYLYSIA (component name: amorphous silicon dioxide, registered trademark of Fuji Silysia Chemical Ltd), and product name: ALCAMAC (component name: synthetic hydrotalcite, registered trademark of Kyowa Chemical Industry Co., Ltd.).

The above-listed adsorbents may be used alone or in combination.

### -Lubricants-

The lubricants are not particularly limited, and may be appropriately selected according to the intended purpose. Examples of the lubricants include, but are not limited to, magnesium stearate, calcium stearate, sucrose fatty acid ester, sodium stearyl fumarate, stearic acid, polyethylene glycol, and talc.

The above-listed lubricants may be used alone or in combination.

### -Odor-masking agents-

The odor-masking agents are not particularly limited, and may be appropriately selected according to the intended purpose. Examples of the odor-masking agents include, but are not limited to, trehalose, malic acid, maltose, potassium gluconate, aniseed essential oil, vanilla essential oil, and cardamom oil.

The above-listed odor-masking agents may be used alone or in combination.

### -Surfactants-

The surfactants are not particularly limited, and may be appropriately selected according to the intended purpose. Examples of the surfactants include, but are not limited to, polysorbate (e.g., polysorbate 80), a polyoxyethylene-polyoxypropylene copolymer, and sodium lauryl sulfate.

The above-listed surfactants may be used alone or in combination.

### -Fragrance-

The fragrance is not particularly limited, and may be appropriately selected according to the intended purpose. Examples of the fragrance include, but are not limited to, lemon oil, orange oil, and peppermint oil.

The above-listed examples of the fragrance may be used alone or in combination.

### -Colorants-

The colorants are not particularly limited, and may be appropriately selected according to the intended purpose. Examples of the colorants include, but are not limited to, titanium oxide, Food Yellow No. 5, Food Blue No. 2, iron sesquioxide, and yellow iron sesquioxide.

The above-listed colorants may be used alone or in combination.

### -Antioxidants-

The antioxidants are not particularly limited, and may be appropriately selected according to the intended purpose. Examples of the antioxidants include, but are not limited to, sodium ascorbate, L-cysteine, sodium sulfite, and vitamin E.

The above-listed antioxidants may be used alone or in combination.

### -Masking agents-

The masking agents are not particularly limited, and may be appropriately selected according to the intended purpose. Examples of the masking agents include, but are not limited to, titanium oxide.

The above masking agents may be used alone or in combination.

### -Anti-static agents-

The anti-static agents are not particularly limited, and may be appropriately selected according to the intended purpose. Examples of the anti-static agents include, but are not limited to, talc and titanium oxide.

The above-listed anti-static agents may be used alone or in combination.

### -Humectants-

The humectants are not particularly limited, and may be appropriately selected according to the intended purpose. Examples of the humectants include, but are not limited to, polysorbate 80, sodium lauryl sulfate, sucrose fatty acid ester, macrogol, and hydroxypropyl cellulose (HPC).

The above-listed humectants may be used alone or in combination.

A pharmaceutical preparation of the pharmaceutical composition is not particularly limited, and may be appropriately selected according to the intended purpose. Examples of the pharmaceutical preparation include, but are not limited to, colon delivery preparations, lipid microsphere preparations, dry emulsion preparations, self-emulsifying preparations, dry syrup, powder preparations for transnasal administration, powder preparations for pulmonary administration, wax matrix preparations, hydrogel preparations, polymeric micelle preparations, mucoadhesive preparations, gastric floating preparations, liposome preparations, and solid dispersion preparations.

The above-listed pharmaceutical preparations may be used alone or in combination.

Examples of a dosage form of the pharmaceutical composition include, but are not limited to: tablets, capsules, suppositories, and other solid dosage forms; intranasal aerosol and aerosol for pulmonary administration; and liquid medicaments, such as injections, intraocular preparations, endaural preparations, and oral preparations.

An administration route of the pharmaceutical composition is not particularly limited, and may be appropriately selected according to the intended purpose. Examples of the administration route include, but are not limited to, oral administration, nasal administration, rectal administration, vaginal administration, subcutaneous administration, intravenous administration, and pulmonary administration. Among the above-listed examples, oral administration is preferable.

### <Functional food>

The functional food in association with the present disclosure includes the particles obtained by the above-described method for producing the particles, and may further include other additives as necessary.

The food is not particularly limited, and may be appropriately selected according to the intended purpose. Examples of the food include, but are not limited to, frozen desserts, noodles, confectioneries, marine products, processed marine and livestock products, dairy products, fats and oils, processed fats and oils, seasonings, retort pouch foods, health foods, and nutritional supplements.

### <Functional cosmetics>

The functional cosmetics in association with the present disclosure include the particles obtained by the above-described method for producing the particles, and may further include other additives as necessary.

The cosmetics are not particularly limited, and may be appropriately selected according to the intended purpose. Examples of the cosmetics include, but are not limited to, skin care products, make-up products, hair care products, body care products, and fragrance products.

### Examples

The present disclosure will be specifically described through Examples and Comparative Examples, but the present disclosure is not limited to Examples in any way.

### <Production example of Particles 1A-1 (first embodiment, liquid column resonance)>

Horseradish peroxidase (HRP, product code: PEO-131, manufactured by TOYOBO CO., LTD.) serving as a bioactive substance and lactose hydrate (product code: 124-00092, manufactured by FUJIFILM Wako Pure Chemical Corporation) serving as a base material were both added to pure water serving as a solvent, to thereby prepare an aqueous solution having an HRP content of 0.97 mg/mL and a lactose content of 0.03 mg/mL. The prepared mixture solution had a solid content of 1 percent by mass.

Then, the obtained mixture solution was passed through a filter (CES-005-M47DK) having a pore diameter of 0.45 µm, to thereby obtain a particle material mixture solution 1A. The obtained particle material mixture solution 1A was discharged as droplets into a gas using a liquid-column-resonance droplet discharger, thereby obtaining Particles 1A-1. The production conditions were as follows.

### -Particle production conditions-

Shape of discharge port: perfect circle
Diameter of discharge port: 8.0 µm
Temperature of conveying gas stream: 40°C
Flow rate of drying gas stream (dry nitrogen): 50 L/min

### <Production Example of Particles 1A-2 (first embodiment, Rayleigh breakup)>

The obtained particle material mixture solution 1A was discharged as droplets into a gas using a Rayleigh breakup discharger thereby obtaining Particles 1A-2. The production conditions were as follows.

### -Particle production conditions-

Shape of discharge port: perfect circle
Diameter of discharge port: 5.0 µm
Temperature of conveying gas stream: 40°C
Flow rate of conveying gas stream: 50 m³/h
Pressure for discharging the mixture solution: 0.4 MPa

### <Production Example of Particles 1A-3 (second embodiment, Rayleigh breakup)>

The obtained particle material mixture solution 1A was discharged as droplets into a vacuum using a Rayleigh breakup discharger, thereby obtaining Particles 1A-3. The production conditions are as follows.

### -Particle production conditions-

Shape of discharge port: perfect circle
Diameter of discharge port: 5.0 µm
Flow rate of drying gas stream: no drying gas stream
Pressure for discharging the mixture solution: 0.4 MPa
Degree of vacuum in chamber: 10 Pa
Cooling jacket temperature: -20°C

### -Freeze drying conditions-

Pre-freezing: -20°C, 30 minutes
Primary freezing: -20°C, 6 hours
Secondary freezing: 10°C, 24 hours

### <Production Example of Particles 1A-4 (third embodiment, Rayleigh breakup)>

The obtained particle material mixture solution 1A was discharged as droplets into a liquefied gas using a Rayleigh breakup discharger, thereby obtaining Particles 1A-4. The production conditions were as follows.

### -Particle production conditions-

Shape of discharge port: perfect circle
Diameter of discharge port: 5.0 µm
Temperature of conveying gas stream: 20°C
Flow rate of conveying gas stream: 50 m³/h
Liquefied gas: liquid nitrogen

### -Freeze drying conditions-

Pre-freezing: -20°C, 30 minutes
Primary freezing: -20°C, 6 hours
Secondary freezing: 10°C, 24 hours

### <Production Example of Particles 1A-5>

Horseradish peroxidase (HRP, product code: PEO-131, manufactured by TOYOBO CO., LTD.) serving as a bioactive substance and polyvinyl alcohol (PVA, product code: 165-17915, manufactured by FUJIFILM Wako Pure Chemical Corporation) serving as a base material were both added to water serving as a solvent to thereby obtain a solution having an HRP content of 0.5 percent by mass and a PVA content of 0.5 percent by mass. The resultant solution was dripped into acetonitrile using a syringe (with syringe needle 25G, manufactured by Terumo Corporation) to thereby obtain a dispersion liquid in which HRP and PVA were formed into particles. The resultant dispersion liquid was vacuum-dried, thereby obtaining Particles 1A-5.

### -Vacuum drying conditions-

Degree of vacuum: 10 Pa
Drying temperature: 20°C
Drying time: 24 hours

### <Production Example of Particles 2A-1 (first embodiment, liquid column resonance)>

An anti-rabbit IgG antibody produced in goat (polyclonal, available from Sigma-Aldrich, product code: R5506) serving as a bioactive substance was added to pure water serving as a solvent to thereby prepare an aqueous solution in which the amount of the anti-rabbit IgG antibody produced in goat was 1.0 mg/mL.

Then, the obtained mixture solution was passed through a filter (CES-005-M47DK) having an average pore diameter of 0.45 µm to thereby obtain a particle material mixture solution 2A. Particles 2A-1 were obtained in the same manner as in <Production Example of Particles 1A-1 (first embodiment, liquid column resonance)>, except that the particle material mixture solution 2A was used instead of the particle material mixture solution 1A.

### <Production Example of Particles 2A-2 (first embodiment, Rayleigh breakup)>

Particles 2A-2 were obtained in the same manner as in <Production Example of Particles 1A-2 (first embodiment, liquid column resonance)>, except that the particle material mixture solution 2A was used instead of the particle material mixture solution 1A.

### <Production Example of Particles 2A-3 (second embodiment, Rayleigh breakup)>

Particles 2A-3 were obtained in the same manner as in <Production Example of Particles 1A-3 (first embodiment, liquid column resonance)>, except that the particle material mixture solution 2A was used instead of the particle material mixture solution 1A.

### <Production Example Particles 2A-4 (third embodiment, Rayleigh breakup)>

Particles 2A-4 were obtained in the same manner as in <Production Example of Particles 1A-4 (first embodiment, liquid column resonance)>, except that the particle material mixture solution 2A was used instead of the particle material mixture solution 1A.

### <Production Example of Particles 3A-1 (first embodiment, liquid column resonance)>

An anti-rabbit IgG antibody produced in goat (polyclonal, Sigma-Aldrich, product code: R5506) serving as a bioactive substance was added to pure water serving as a solvent A to thereby prepare a liquid A1. The liquid A1 was prepared so that the amount of the bioactive substance relative to the volume of the liquid A1 was 50.0 mg/mL.

Sorbitan sesquioleate (SPAN83, manufactured by Tokyo Chemical Industry Co., Ltd.) serving as a surfactant was added to dichloromethane (manufactured by FUJIFILM Wako Pure Chemical Corporation) serving as a solvent B to thereby prepare a liquid B 1. The liquid B1 was prepared so that the amount of the sorbitan sesquioleate relative to the total amount of the liquid B1 was 0.1 percent by mass.

Next, the liquid A1 and the liquid B1 were mixed so that a mass ratio of the liquid A1 to the liquid B1 (liquid A1 : liquid B1) was 5:95. The solution mixture was homogenized by a homogenizer (device name: sonicstar85, manufactured by AS ONE Corporation) at the output of 90% for 10 minutes to prepare a dispersion liquid 1 (W/O emulsion), thereby obtaining a particle material mixture solution 3A. The size of the droplets of the liquid A1 in the dispersion liquid was measured by a concentration system analyzer ("FPAR-1000" manufactured by Otsuka Electronics Co., Ltd.) according to dynamic light scattering. As a result, the size of the droplets was 450 nm. Particles 3A-1 were obtained in the same manner as in <Production Example of Particles 1A-1 (first embodiment, liquid column resonance)>, except that the particle material mixture solution 3A was used instead of the particle material mixture solution 1A.

### <Production Example of Particles 3A-2 (first embodiment, Rayleigh breakup)>

Particles 3A-2 were obtained in the same manner as in <Production Example of Particles 1A-2 (first embodiment, liquid column resonance)>, except that the particle material mixture solution 3A was used instead of the particle material mixture solution 1A, and the temperature of the conveying gas stream was changed to 30°C.

### <Production Example of Particles 3A-3 (second embodiment, Rayleigh breakup)>

Particles 3A-3 were obtained in the same manner as in <Production Example of Particles 1A-3 (first embodiment, liquid column resonance)>, except that the particle material mixture solution 3A was used instead of the particle material mixture solution 1A, and the temperature of the conveying gas stream was changed to 30°C.

### <Production Example of Particles 3A-4 (third embodiment, Rayleigh breakup)>

Particles 3A-4 were obtained in the same manner as in <Production Example of Particles 1A-4 (first embodiment, liquid column resonance)>, except that the particle material mixture solution 3A was used instead of the particle material mixture solution 1A.

### <Production Example of Particles 3A-5 (spray nozzle)>

Particles 3A-5 were obtained from the particle material mixture solution 3A using a spray nozzle (Tri Spire Nozzle, manufactured by GF).

### -Particle production conditions-

Dryer size: 150 mm in diameter, 500 mm in height
Feeding rate of the mixture solution (discharging condition of Tri Spire Nozzle): 10 g/min
Amount of compressed air (discharging condition of Tri Spire Nozzle): 0.6 MPa, 40 NL/min
Flow rate of conveying gas stream: 50 m³/h
Temperature of drying gas stream: 30°C

### <Production Example of Particles 2B>

Particles 2B were obtained from the particle material mixture solution 2A using a spray dryer (rotary disk atomizer, manufactured by OHKAWARA KAKOHKI CO., LTD.).

### -Particle production conditions-

Spray dryer model: L-8
Rotational speed of the rotary disk atomizer: 10,000 rpm
Feeding rate of the mixture solution: 2 kg/h
Flow rate of conveying gas stream: 50 m³/h
Temperature of drying gas stream: 80°C

### (Examples 1 to 14, and Comparative Example 1)

The following measurement and evaluations were performed on each of the above particles.

The measurement and evaluations performed on Particles 1A-1 to Particles 3A-5 were determined as Examples 1 to 14, respectively, and the measurement and evaluations performed on Particles 2B were determined as Comparative Example 1.

### [Quantification of amount of bioactive substance (HRP)]

A calibration curve representing the relationship between the concentration of the horseradish peroxidase (HRP, PEO-131, available from TOYOBO CO., LTD.) dissolved in pure water and the absorbance at the measurement wavelength of 400 nm was prepared. For the measurement of the absorbance, a microsample spectrophotometer (SimpliNano, manufactured by GE HealthCare) was used.

Next, the absorbance of each of Particles 1A-1 to 1A-5 at the measurement wavelength of 400 nm was measured, and the amount of HRP contained in each of Particles 1A-1 to 1A-5 was calculated based on the prepared calibration curve. The results are presented in Tables 1-1 and 1-2. The numerical values of the amounts presented in Tables 1-1 and 1-2 are numerical values when the value (theoretical value) in the case where the whole amount of HRP in the particle material mixture solution 1A is incorporated into the particles after the production is determined as 100%.

### [Quantification of bioactive substance (HRP) activity]

The HRP activity of each of Particles 1A-1 to 1A-5 was evaluated based on the protocol of the kit (Pierce TMB Substrate Kit, manufactured by Thermo Fisher Scientific Inc., product code: 34021). The device and reagents used were as follows.

### -Used devices-

Microplate photospectrometer Maltiskan GO (manufactured by Thermo Fisher Scientific Inc.)
Nunc Edge 2.0 96-Well Plates (product code: 167425, manufactured by Thermo Fisher Scientific Inc.)

### -Used reagents-

Horseradish peroxidase (HRP, product code: PEO-131, available from TOYOBO CO., LTD.)
Hydroxypropyl cellulose (HPC 2.0 to 2.9, product code: 082-07925, available from FUJIFILM Wako Pure Chemical Corporation)
Pierce TMB Substrate Kit (product code: 34021, manufactured by Thermo Fisher Scientific Inc.)

The results are presented in Tables 1-1 and 1-2 below. The numerical values of the amounts presented in Tables 1-1 and 1-2 are numerical values when the value (theoretical value) in the case where the whole amount of HRP in the particle material mixture solution 1A exhibits activity in the particles after the production is determined as 100%.

### [Quantification of amount of bioactive substance (anti-rabbit IgG antibody produced in goat)]

A calibration curve representing the relationship between the concentration of the anti-rabbit IgG antibody produced in goat (polyclonal, product code: R5506, Sigma-Aldrich) dissolved in pure water and the absorbance at the measurement wavelength of 280 nm was prepared. For the measurement of the absorbance, a microsample spectrophotometer (SimpliNano, manufactured by GE HealthCare) was used.

Next, the absorbance of each of Particles 2A-1 to 3A-5 and Particles 2B at the measurement wavelength of 280 nm was measured, and the amount of the anti-rabbit IgG antibody produced in goat contained in each of Particles 2A-1 to 3A-5 and Particles 2B was calculated based on the prepared calibration curve. The results are presented in Tables 1-1 and 1-2. The numerical values of the amounts presented in Tables 1-1 and 1-2 are numerical values when the value (theoretical value) in the case where the whole amount of the anti-rabbit IgG antibody produced in goat contained in the particle material mixture solution 2A is incorporated into the particles after the production is determined as 100%.

### [Quantification of bioactive substance (anti-rabbit IgG antibody produced in goat) activity]

The activity of the anti-rabbit IgG antibody produced in goat in each of Particles 2A-1 to 3A-5 and Particles 2B was evaluated by ELISA (sandwich method). The ELISA (sandwich method) was carried out according to a commonly used method, and the device and reagents used were as follows.

### -Used device-

Microplate photospectrometer Maltiskan GO (manufactured by Thermo Fisher Scientific Inc.)
Nunc MaxiSorp flat-bottom (product code: 44-2404-21, manufactured by Thermo Fisher Scientific Inc.)

### -Used reagents-

Primary antibody: anti-rabbit IgG antibody produced in goat (polyclonal, product code: R5506, available from Sigma-Aldrich)
Antigen: IgG from rabbit serum (product code: I5006, available from Sigma-Aldrich)
Secondary antibody: anti-rabbit IgG antibody produced in goat, peroxidase-labeled (polyclonal, product code: A0545, available from Sigma-Aldrich)
Hydroxypropyl cellulose (HPC 2.0 to 2.9, product code: 082-07925, available from FUJIFILM Wako Pure Chemical Corporation)
Bovine serum albumin (BSA) (pH 7.0, product code: 019-23293, available from FUJIFILM Wako Pure Chemical Corporation)
Polyoxyethylene(20)sorbitan monolaurate (product code: 166-21213, available from FUJIFILM Wako Pure Chemical Corporation)
Pierce TMB Substrate Kit (product code: 34021, available from Thermo Fisher Scientific Inc.)

### [Quantification of viscosity µ1/viscosity µ0]

The viscosity ratio (viscosity µ1/viscosity µ0) was calculated where µ1 was the viscosity when each of the particles were dispersed in MCT oil (COCONAD RK, manufactured by Kao Corporation) at the concentration of 400 mg/mL, and µ0 was the viscosity when the particles were not included in the MCT oil. The results are presented in Tables 1-1 and 1-2.

**Table 1-1**

| | | Production conditions | | | |
|---|---|---|---|---|---|
| | | Bioactive substance | Base material | Discharge method | Discharging step |
| Ex. 1 | Particles 1A-1 | HRP | lactose | liquid column resonance | discharging in gas |
| Ex. 2 | Particles 1A-2 | HRP | lactose | Rayleigh breakup | discharging in gas |
| Ex. 3 | Particles 1A-3 | HRP | lactose | Rayleigh breakup | discharging in vacuum |
| Ex. 4 | Particles 1A-4 | HRP | lactose | Rayleigh breakup | discharging in liquefied gas |
| Ex. 5 | Particles 1A-5 | HRP | PVA | syringe | dripping in acetonitrile |
| Ex. 6 | Particles 2A-1 | IgG | N/A | liquid column resonance | discharging in gas |
| Ex. 7 | Particles 2A-2 | IgG | N/A | Rayleigh breakup | discharging in gas |
| Ex. 8 | Particles 2A-3 | IgG | N/A | Rayleigh breakup | discharging in vacuum |
| Ex. 9 | Particles 2A-4 | IgG | N/A | Rayleigh breakup | discharging in liquefied gas |
| Ex. 10 | Particles 3A-1 | IgG | N/A | liquid column resonance | discharging in gas |
| Ex. 11 | Particles 3A-2 | IgG | N/A | Rayleigh breakup | discharging in gas |
| Ex. 12 | Particles 3A-3 | IgG | N/A | Rayleigh breakup | discharging in vacuum |
| Ex. 13 | Particles 3A-4 | IgG | N/A | Rayleigh breakup | discharging in liquefied gas |
| Ex. 14 | Particles 3A-5 | IgG | N/A | spray nozzle | discharging in gas |
| Comp. Ex. 1 | Particles 2B | IgG | N/A | rotary atomizer | discharging in gas |

**Table 1-2 (continued from Table 1-1)**

| | | Physical properties of particles | | | | |
|---|---|---|---|---|---|---|
| | | Particle size (µm) | R.S.F. | Amount of bioactive substance (%) | Activity of bioactive substance (%) | µ1/µ0 |
| Ex. 1 | Particles 1A-1 | 2.1 | 0.7 | 102 | 82 | 4.4 |
| Ex. 2 | Particles 1A-2 | 4.2 | 0.8 | 97 | 81 | 3.6 |
| Ex. 3 | Particles 1A-3 | 5.4 | 0.9 | 99 | 90 | 3.3 |
| Ex. 4 | Particles 1A-4 | 4.8 | 1.2 | 101 | 94 | 3.4 |
| Ex. 5 | Particles 1A-5 | 0.11 | 1.2 | 96 | 81 | 11.0 |
| Ex. 6 | Particles 2A-1 | 2.4 | 0.6 | 99 | 85 | 4.2 |
| Ex. 7 | Particles 2A-2 | 4.4 | 0.8 | 96 | 83 | 3.6 |
| Ex. 8 | Particles 2A-3 | 4.9 | 1.0 | 98 | 94 | 3.3 |
| Ex. 9 | Particles 2A-4 | 5.1 | 1.1 | 96 | 92 | 3.4 |
| Ex. 10 | Particles 3A-1 | 2.3 | 0.6 | 99 | 90 | 3.9 |
| Ex. 11 | Particles 3A-2 | 4.1 | 0.7 | 99 | 91 | 3.8 |
| Ex. 12 | Particles 3A-3 | 5.1 | 1.1 | 99 | 93 | 3.3 |
| Ex. 13 | Particles 3A-4 | 5.0 | 1.1 | 97 | 91 | 3.3 |
| Ex. 14 | Particles 3A-5 | 2.5 | 1.3 | 98 | 94 | 4.0 |
| Comp. Ex. 1 | Particles 2B | 15.2 | 1.8 | 98 | 13 | 3.0 |

Embodiments of the present disclosure are, for example, as follows.
<1> A method for producing particles, containing:
   preparing a bioactive-substance-containing liquid that includes a bioactive substance and a solvent;
   discharging the bioactive-substance-containing liquid as droplets; and
   removing the solvent from the droplets to form particles,
   wherein a biological activity ratio represented by {(biological activity level B/biological activity level A) × 100} is 80% or greater, where the biological activity level A is a biological activity level of the bioactive substance before the preparing the bioactive-substance-containing liquid, and the biological activity level B is a biological activity level of the bioactive substance after the preparing of the bioactive-substance-containing liquid.
<2> The method according to <1>,
   wherein external stress is not applied to the bioactive-substance-containing liquid and the droplets.
<3> The method according to <1> or <2>,
   wherein the discharging is discharging the bioactive-substance-containing liquid into a gas.
<4> The method according to <1> or <2>,
   wherein the discharging is discharging the bioactive-substance-containing liquid into a vacuum.
<5> The method according to <1> or <2>,
   wherein the discharging is discharging the bioactive-substance-containing liquid into a liquefied gas.
<6> The method according to any one of <1> to <3>,
   wherein the discharging is discharging the bioactive-substance-containing liquid from one or more discharge holes using vibration.
<7> The method according to any one of <1> and <3> to <6>,
   wherein the bioactive-substance-containing liquid is a water-in-oil emulsion,
      and
   diameters of droplets of the bioactive-substance-containing liquid in the water-in-oil emulsion are 100 nm or greater and 5 µm or less.
<8> The method according to <7>,
   wherein the water-in-oil emulsion includes a surfactant having a hydrophilic-lipophilic balance of 1 or greater and 8 or less.
<9> The method according to <8>,
   wherein the surfactant is sorbitan sesquioleate.
<10> Particles produced by the method according to any one of <1> to <9>,
   wherein the particles have a volume average particle diameter of 1 µm or greater and 50 µm or less, and
   the particles have a relative span factor of 1.5 or less.
<11> The particles according to <10>,
   wherein the bioactive substance includes a protein, a nucleic acid, or both a protein and a nucleic acid.
<12> The particles according to <10> or <11>,
   wherein the bioactive substance includes an antibody, an enzyme, or both an antibody and an enzyme.
<13> The particles according to any one of <10> to <12>,
   wherein an amount of the bioactive substance is 95 percent by mass or greater relative to a total amount of the particles.
<14> A pharmaceutical composition containing:
   the particles according to any one of <10> to <13>; and
   a lipid.
<15> The pharmaceutical composition according to <14>,
   wherein a ratio µ1/µ0 of a viscosity µ1 to a viscosity µ0 is 10 or less, where the viscosity µ1 is a viscosity of the pharmaceutical composition in which an amount of the particles is 400 mg/mL, and the viscosity µ0 is a viscosity of the pharmaceutical composition in which the amount of the particles is 0 mg/mL.
<16> The pharmaceutical composition according to <14> or <15>,
   wherein the lipid is soybean oil, olive oil, fish oil, sesame oil, canola oil, sunflower oil, castor oil, coconut oil, palm oil, corn oil, or medium-chain triglyceride.
<17> The pharmaceutical composition according to any one of <14> to <16>,
   wherein an amount of the particles dispersed in the lipid is 1 mg/mL or greater and 500 mg/mL or less.
<18> The pharmaceutical composition according to any one of <14> to <17>, further including:
   an excipient.

The method for producing particles according to any one of <1> to <9>, the particles according to any one of <10> to <13>, and the pharmaceutical composition according to any one of <14> to <18> can solve the various problems existing in the related art, and can achieve the object of the present disclosure.

Althrough the embodiments of the present invention have been described above, the present invention is not limited to these embodiments. Various variations and modifications may be made without departing from the scope of the present invention.

## Claims

1. A method for producing particles, comprising:
preparing a bioactive-substance-containing liquid that includes a bioactive substance and a solvent;
discharging the bioactive-substance-containing liquid as droplets; and
removing the solvent from the droplets to form particles,
wherein a biological activity ratio represented by {(biological activity level B/biological activity level A) × 100} is 80% or greater, where the biological activity level A is a biological activity level of the bioactive substance before the preparing the bioactive-substance-containing liquid, and the biological activity level B is a biological activity level of the bioactive substance after the preparing of the bioactive-substance-containing liquid.

2. The method according to claim 1,
wherein external stress is not applied to the bioactive-substance-containing liquid and the droplets.

3. The method according to claim 1 or 2,
wherein the discharging is discharging the bioactive-substance-containing liquid into a gas, a vacuum, or a liquefied gas.

4. The method according to claim 1 or 2,
wherein the discharging is discharging the bioactive-substance-containing liquid from one or more discharge holes using vibration.

5. The method according to claim 1,
wherein the bioactive-substance-containing liquid is a water-in-oil emulsion,
and
diameters of droplets of the bioactive-substance-containing liquid in the water-in-oil emulsion are 100 nm or greater and 5 µm or less.

6. The method according to claim 5,
wherein the water-in-oil emulsion includes a surfactant having a hydrophilic-lipophilic balance of 1 or greater and 8 or less.

7. The method according to claim 6,
wherein the surfactant is sorbitan sesquioleate.

8. Particles produced by the method according to claim 1 or 2,
wherein the particles have a volume average particle diameter of 1 µm or greater and 50 µm or less, and
the particles have a relative span factor of 1.5 or less.

9. The particles according to claim 8,
wherein the bioactive substance includes at least one selected from the group consisting of a protein, a nucleic acid, an antibody, and an enzyme.

10. The particles according to claim 8,
wherein an amount of the bioactive substance is 95 percent by mass or greater relative to a total amount of the particles.

11. A pharmaceutical composition comprising:
the particles according to claim 8; and
a lipid.

12. The pharmaceutical composition according to claim 11,
wherein a ratio µ1/µ0 of a viscosity µ1 to a viscosity µ0 is 10 or less, where the viscosity µ1 is a viscosity of the pharmaceutical composition in which an amount of the particles is 400 mg/mL, and the viscosity µ0 is a viscosity of the pharmaceutical composition in which the amount of the particles is 0 mg/mL.

13. The pharmaceutical composition according to claim 11,
wherein the lipid is soybean oil, olive oil, fish oil, sesame oil, canola oil, sunflower oil, castor oil, coconut oil, palm oil, corn oil, or medium-chain triglyceride.

14. The pharmaceutical composition according to claim 11,
wherein an amount of the particles dispersed in the lipid is 1 mg/mL or greater and 500 mg/mL or less.

15. The pharmaceutical composition according to claim 11, further comprising:
an excipient.
